(19) Europäisches Patentamt
European Patent Office
Office européen des brevets

(11) EP 4 183 799 A1

(12) EUROPEAN PATENT APPLICATION
published in accordance with Art. 153(4) EPC

(43) Date of publication:
**24.05.2023 Bulletin 2023/21**

(21) Application number: **21841453.0**

(22) Date of filing: **16.07.2021**

(51) International Patent Classification (IPC):
***C07K 16/00*** *(2006.01)*

(52) Cooperative Patent Classification (CPC):
**C07K 16/00**

(86) International application number:
**PCT/JP2021/026784**

(87) International publication number:
**WO 2022/014698 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2020 JP 2020122695**

(71) Applicant: **Daiichi Sankyo Company, Limited**
**Tokyo 103-8426 (JP)**

(72) Inventors:
- **SATO, Hitoshi**
  **Tokyo 103-8426 (JP)**
- **SASORI, Yuka**
  **Tokyo 103-8426 (JP)**
- **FUKATSU, Daisuke**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) METHOD FOR PRODUCING ANTIBODY-DRUG CONJUGATE

(57) A method for producing an antibody-drug conjugate composition, comprising: (i) a step of reacting an antibody with a reducing agent to obtain an antibody having thiol groups; then (ii) a step of reacting drug-linker intermediates with the antibody having thiol groups obtained in the step (i), wherein the step (i) is carried out until the composition ratio of the antibody having four heavy-light interchain thiols and the composition ratio of the antibody having four heavy-heavy interchain thiols reach a steady value.

## Description

### Technical Field

The present invention relates to a method for stably producing an antibody-drug conjugate composition comprising each of the isomers of the antibody-drug conjugate having different binding positions of the drug in a constant ratio.

### Background Art

An antibody-drug conjugate (ADC) having a drug with cytotoxicity conjugated to an antibody which binds to an antigen expressed on the surface of cancer cells and is also capable of cellular internalization can deliver the drug selectively to cancer cells, and is thus expected to cause accumulation of the drug within cancer cells and to kill the cancer cells (Non-Patent References 1 to 5).

In an antibody, there are four interchain disulfides, which are readily reduced because they are more likely to come close to solvent than other disulfides, and each of them can be used as a bonding site to a drug (or a drug-linker) in an antibody-drug conjugate. An antibody-drug conjugate with up to eight drug molecules conjugated per antibody molecule is produced by reducing interchain disulfides in an antibody and conjugating drug molecules to the thiol groups generated.

Antibody-drug conjugates with eight drug molecules conjugated per antibody molecule may cause less tolerability in terms of safety, even if they are excellent in antitumor effect. For this reason, antibody-drug conjugates with an average number of conjugated drug molecules of less than eight are used in some cases to improve tolerability in terms of safety with retaining therapeutic efficacy. Antibody-drug conjugates with an average number of conjugated drug molecules of less than eight can be obtained, for example, by reacting with control of the amount of drug per antibody molecule; however, the reaction product is a composition of antibody-drug conjugates with numbers of conjugated drug molecules of 2, 4, 6, and 8. Accordingly, even when antibody-drug conjugate compositions have the same average number of conjugated drug molecules, they may be different in therapeutic efficacy and tolerability in terms of safety if they differ in the distribution of numbers of conjugated drug molecules. In the case of antibody-drug conjugate compositions with an average number of conjugated drug molecules of about 4, for example, those having high contents of antibody-drug conjugates with numbers of conjugated drug molecules of 0 and 8 may have lower therapeutic efficacy and cause less tolerability in terms of safety than those having a high content of antibody-drug conjugates with a number of conjugated drug molecules of 4. Moreover, even antibody-drug conjugates having the same number of conjugated drug molecules may be different in therapeutic efficacy and tolerability in terms of safety depending on the binding positions of the drug. Therefore, in producing an antibody-drug conjugate composition, a method for producing an antibody-drug conjugate composition with the number of conjugated drug molecules and binding positions thereof controlled is demanded.

Known examples of methods for producing an antibody-drug conjugate composition with the number of conjugated drug molecules and binding positions thereof controlled include: a method of selectively producing an antibody-drug conjugate in which four units of a drug-linker are conjugated to heavy-heavy interchain thiols by temporarily reducing the interchain disulfides in an antibody completely, returning some of the interchain thiols generated to disulfides through re-oxidation, and conjugating drug molecules to the residual interchain thiols (Patent Reference 1); and a method of selectively producing an antibody-drug conjugate in which four units of a drug-linker are conjugated to heavy-light interchain thiols by reducing interchain disulfides in an antibody at low temperature and conjugating drug molecules to the interchain thiols generated (Patent Reference 2).

### Citation List

#### Patent Literature

Patent Reference 1: International Publication No. 2005/084390
Patent Reference 2: International Publication No. 2017/002776

#### Non-Patent Literature

Non-Patent Reference 1: Ducry, L., et al., Bioconjugate Chem. (2010) 21, 5-13.

Non-Patent Reference 2: Alley, S. C., et al., Current Opinion in Chemical Biology (2010) 14, 529-537.
Non-Patent Reference 3: Damle N. K. Expert Opin. Biol. Ther. (2004) 4, 1445-1452.
Non-Patent Reference 4: Senter P. D., et al., Nature Biotechnology (2012) 30, 631-637.
Non-Patent Reference 5: Howard A. et al., J Clin Oncol 29: 398-405.

## Summary of Invention

### Technical Problem

Methods for producing an antibody-drug conjugate composition containing a specific isomer, among isomers of an antibody-drug conjugate having different binding positions of a drug, in a high ratio are known (International Publication No. 2005/084390 and International Publication No. 2017/002776). However, a method for stably producing an antibody-drug conjugate composition comprising each of the isomers in a constant ratio, rather than containing a specific isomer in a high ratio, may be demanded from an industrial viewpoint. Such a method for producing an antibody-drug conjugate composition is not known, and development of an industrially excellent production method has been demanded.

### Solution to Problem

As a result of diligent studies in order to solve the above problems, the present inventors have found that by carrying out a step of reacting an antibody with a reducing agent to obtain an antibody having thiol groups for a long time, the composition ratio of the antibody having four heavy-light interchain thiols and the composition ratio of the antibody having four heavy-heavy interchain thiols reach a steady value (hereinafter, also referred to as "equilibration of reduction reaction"). In addition, the present inventors have found that by reacting drug-linker intermediates with the thus-obtained antibody having thiol groups, an antibody-drug conjugate composition comprising each of the isomers of the antibody-drug conjugate having different binding positions of the drug in a constant ratio can be stably produced, thus completing the present invention.
Thus, the present invention provides the following [1] to [56] .

[1] A method for producing an antibody-drug conjugate composition, comprising:

(i) a step of reacting an antibody with a reducing agent to obtain an antibody having thiol groups; then
(ii) a step of reacting drug-linker intermediates with the antibody having thiol groups obtained in the step (i), wherein

the step (i) is carried out until the composition ratio of the antibody having four heavy-light interchain thiols and the composition ratio of the antibody having four heavy-heavy interchain thiols reach a steady value.
[2] The production method according to [1], wherein the step (i) is carried out for 4 hours or more.
[3] The production method according to [1], wherein the step (i) is carried out for 12 hours or more.
[4] The production method according to [1], wherein the step (i) is carried out for 16 hours or more.
[5] The production method according to [1], wherein the step (i) is carried out for 20 hours or more.
[6] The production method according to [1], wherein the step (i) is carried out for 31 hours or more.
[7] The production method according to any one of [1] to [6], wherein the step (i) is carried out at 0 to 20°C.
[8] The production method according to any one of [1] to [6], wherein the step (i) is carried out at 5 to 20°C.
[9] The production method according to any one of [1] to [6], wherein the step (i) is carried out at 5 to 10°C.
[10] The production method according to any one of [1] to [6], wherein the step (i) is carried out at about 10°C.
[11] The production method according to any one of [1] to [10], wherein the average number of units of the drug-linker conjugated per antibody molecule in the produced antibody-drug conjugate composition is in the range of from 3.5 to 4.5.
[12] The production method according to any one of [1] to [11], wherein the content of the antibody-drug conjugates in which four drug-linkers are conjugated, in the produced antibody-drug conjugate composition is 50% or more.
[13] The production method according to any one of [1] to [12], wherein the composition ratio of the antibody-drug conjugates in which four drug-linkers are conjugated to heavy-light interchain thiols is 1.5 to 2.5 times the composition ratio of the antibody-drug conjugates in which four drug-linkers are conjugated to heavy-heavy interchain thiols in the produced antibody-drug conjugate composition.
[14] The production method according to any one of [1] to [13], wherein the reducing agent is used at 1.9 to 2.5 equivalents per molecule of antibody.
[15] The production method according to any one of [1] to [14], wherein the reducing agent is tris(2-carboxyethyl)phosphine or a salt thereof.

[16] The production method according to any one of [1] to [15], wherein the drug-linker intermediate has an N-substituted maleimidyl group.

[17] The production method according to any one of [1] to [15], wherein the drug-linker intermediate is a compound represented by the following formula:

[Formula 1]

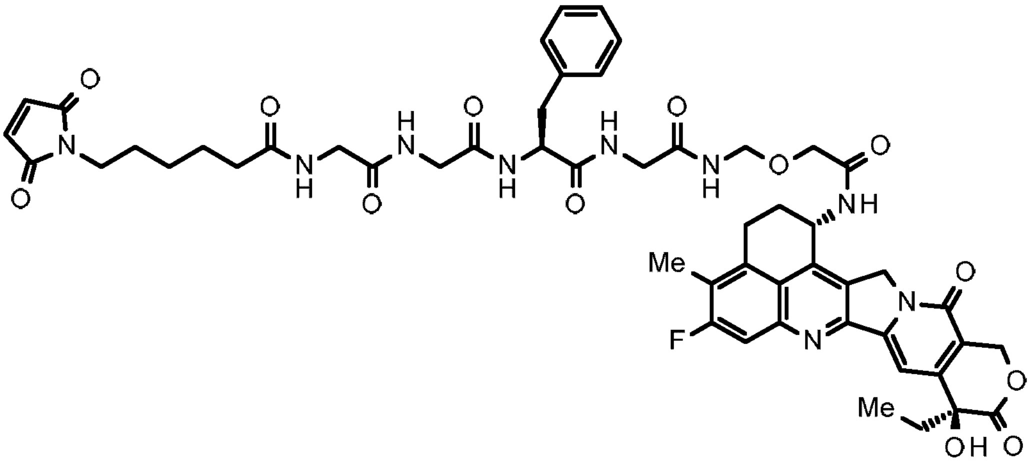

[18] The production method according to [17], wherein the drug-linker in the produced antibody-drug conjugate composition is represented by the following formula:

[Formula 2]

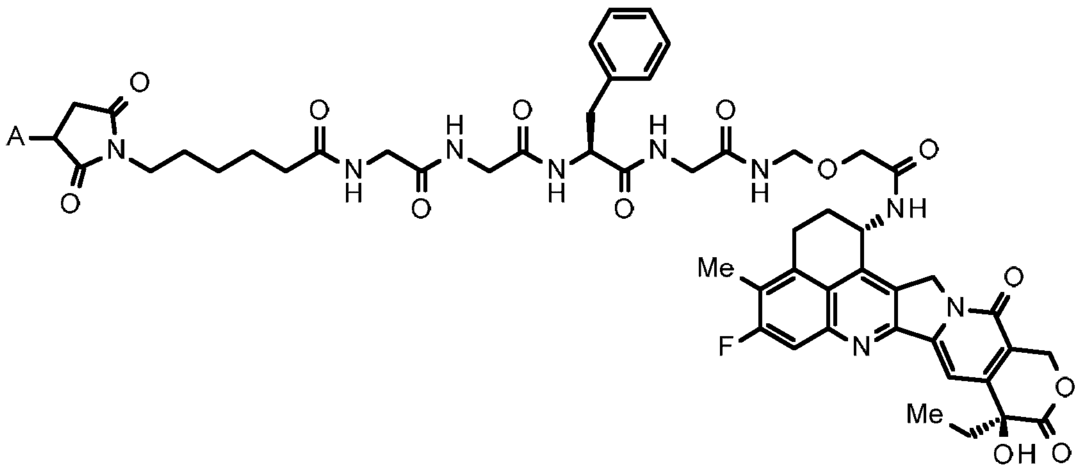

wherein A represents the connecting position to the antibody, and the drug-linker is conjugated to the antibody via a thioether bond.

[19] The production method according to any one of [1] to [18], wherein the antibody is an anti-TROP2 antibody or an anti-B7-H3 antibody.

[20] The production method according to [19], wherein the antibody is an anti-TROP2 antibody.

[21] The production method according to [20], wherein the anti-TROP2 antibody is an antibody comprising a heavy chain comprising CDRH1 consisting of an amino acid sequence represented by SEQ ID NO: 5, CDRH2 consisting of an amino acid sequence represented by SEQ ID NO: 6 and CDRH3 consisting of an amino acid sequence represented by SEQ ID NO: 7, and a light chain comprising CDRL1 consisting of an amino acid sequence represented by SEQ ID NO: 8, CDRL2 consisting of an amino acid sequence represented by SEQ ID NO: 9 and CDRL3 consisting of an amino acid sequence represented by SEQ ID NO: 10.

[22] The production method according to [20], wherein the anti-TROP2 antibody is an antibody comprising a heavy

chain comprising a heavy chain variable region consisting of an amino acid sequence consisting of amino acid residues 20 to 140 of SEQ ID NO: 1 and a light chain comprising a light chain variable region consisting of an amino acid sequence consisting of amino acid residues 21 to 129 of SEQ ID NO: 2.
[23] The production method according to [20], wherein the anti-TROP2 antibody is an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2.
[24] The production method according to [23], wherein the anti-TROP2 antibody lacks a lysine residue at the carboxyl terminus of the heavy chain.
[25] The production method according to [19], wherein the antibody is an anti-B7-H3 antibody.
[26] The production method according to [25], wherein the anti-B7-H3 antibody is an antibody comprising a heavy chain comprising CDRH1 consisting of an amino acid sequence represented by SEQ ID NO: 11, CDRH2 consisting of an amino acid sequence represented by SEQ ID NO: 12 and CDRH3 consisting of an amino acid sequence represented by SEQ ID NO: 13, and a light chain comprising CDRL1 consisting of an amino acid sequence represented by SEQ ID NO: 14, CDRL2 consisting of an amino acid sequence represented by SEQ ID NO: 15 and CDRL3 consisting of an amino acid sequence represented by SEQ ID NO: 16.
[27] The production method according to [25], wherein the anti-B7-H3 antibody is an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.
[28] The production method according to [25], wherein the anti-B7-H3 antibody is an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.
[29] The production method according to [28], wherein the anti-B7-H3 antibody lacks a lysine residue at the carboxyl terminus of the heavy chain.
[30] An antibody-drug conjugate composition produced by the production method according to any one of [1] to [29].
[31] A method for producing an antibody-drug conjugate composition, comprising:

(i) a step of reacting an antibody with a reducing agent to obtain an antibody having thiol groups; then
(ii) a step of reacting drug-linker intermediates with the antibody having thiol groups obtained in the step (i), wherein

the step (i) is carried out at 0 to 20°C for 4 hours or more,
the content of the antibody-drug conjugates in which four drug-linkers are conjugated, in the produced antibody-drug conjugate composition is 50% or more, and
the composition ratio of the antibody-drug conjugates in which four drug-linkers are conjugated to heavy-light interchain thiols is 1.5 to 2.5 times the composition ratio of the antibody-drug conjugates in which four drug-linkers are conjugated to heavy-heavy interchain thiols.

[32] The production method according to [31], wherein the step (i) is carried out for 12 hours or more.
[33] The production method according to [31], wherein the step (i) is carried out for 16 hours or more.
[34] The production method according to [31], wherein the step (i) is carried out for 20 hours or more.
[35] The production method according to [31], wherein the step (i) is carried out for 31 hours or more.
[36] The production method according to any one of [31] to [35], wherein the step (i) is carried out at 5 to 20°C.
[37] The production method according to any one of [31] to [35], wherein the step (i) is carried out at 5 to 10°C.
[38] The production method according to any one of [31] to [35], wherein the step (i) is carried out at about 10°C.
[39] The production method according to any one of [31] to [38], wherein the average number of units of the drug-linker conjugated per antibody molecule in the produced antibody-drug conjugate composition is in the range of from 3.5 to 4.5.
[40] The production method according to any one of [31] to [39], wherein the reducing agent is used at 1.9 to 2.5 equivalents per molecule of antibody.
[41] The production method according to any one of [31] to [40], wherein the reducing agent is tris(2-carboxyethyl)phosphine or a salt thereof.
[42] The production method according to any one of [31] to [41], wherein the drug-linker intermediate has an N-substituted maleimidyl group.
[43] The production method according to any one of [31] to [41], wherein the drug-linker intermediate is a compound represented by the following formula:

[Formula 3]

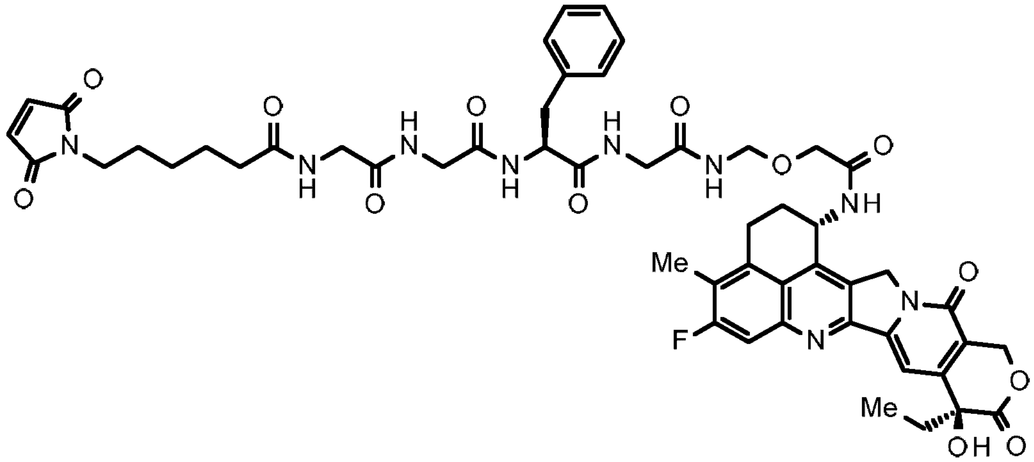

[44] The production method according to [43], wherein the drug-linker in the produced antibody-drug conjugate composition is represented by the following formula:

[Formula 4]

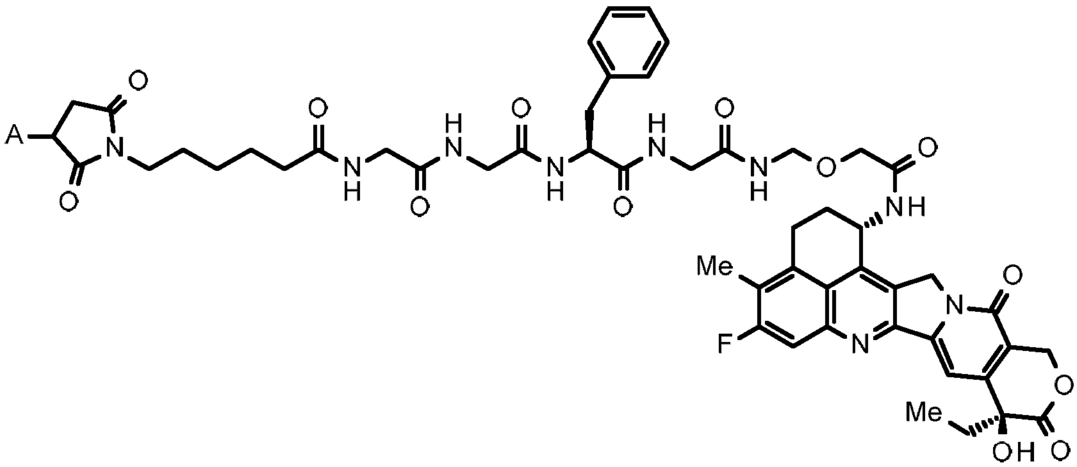

wherein A represents the connecting position to the antibody, and the drug-linker is conjugated to the antibody via a thioether bond.

[45] The production method according to any one of [31] to [44], wherein the antibody is an anti-TROP2 antibody or an anti-B7-H3 antibody.

[46] The production method according to [45], wherein the antibody is an anti-TROP2 antibody.

[47] The production method according to [46], wherein the anti-TROP2 antibody is an antibody comprising a heavy chain comprising CDRH1 consisting of an amino acid sequence represented by SEQ ID NO: 5, CDRH2 consisting of an amino acid sequence represented by SEQ ID NO: 6 and CDRH3 consisting of an amino acid sequence represented by SEQ ID NO: 7, and a light chain comprising CDRL1 consisting of an amino acid sequence represented by SEQ ID NO: 8, CDRL2 consisting of an amino acid sequence represented by SEQ ID NO: 9 and CDRL3 consisting of an amino acid sequence represented by SEQ ID NO: 10.

[48] The production method according to [46], wherein the anti-TROP2 antibody is an antibody comprising a heavy chain comprising a heavy chain variable region consisting of an amino acid sequence consisting of amino acid residues 20 to 140 of SEQ ID NO: 1 and a light chain comprising a light chain variable region consisting of an amino acid sequence consisting of amino acid residues 21 to 129 of SEQ ID NO: 2.

[49] The production method according to [46], wherein the anti-TROP2 antibody is an antibody comprising a heavy chain consisting of an amino acid sequence consisting of amino acid residues 20 to 470 of SEQ ID NO: 1 and a

light chain consisting of an amino acid sequence consisting of amino acid residues 21 to 234 of SEQ ID NO: 2.

[50] The production method according to [49], wherein the anti-TROP2 antibody lacks a lysine residue at the carboxyl terminus of the heavy chain.

[51] The production method according to [45], wherein the antibody is an anti-B7-H3 antibody.

[52] The production method according to [51], wherein the anti-B7-H3 antibody is an antibody comprising a heavy chain comprising CDRH1 consisting of an amino acid sequence represented by SEQ ID NO: 11, CDRH2 consisting of an amino acid sequence represented by SEQ ID NO: 12 and CDRH3 consisting of an amino acid sequence represented by SEQ ID NO: 13, and a light chain comprising CDRL1 consisting of an amino acid sequence represented by SEQ ID NO: 14, CDRL2 consisting of an amino acid sequence represented by SEQ ID NO: 15 and CDRL3 consisting of an amino acid sequence represented by SEQ ID NO: 16.

[53] The production method according to [51], wherein the anti-B7-H3 antibody is an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[54] The production method according to [51], wherein the anti-B7-H3 antibody is an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[55] The production method according to [54], wherein the anti-B7-H3 antibody lacks a lysine residue at the carboxyl terminus of the heavy chain.

[56] An antibody-drug conjugate composition produced by the production method according to any one of [31] to [55].

Advantageous Effects of Invention

The present invention can provide a method for stably producing an antibody-drug conjugate composition comprising each of the isomers of the antibody-drug conjugate having different binding positions of the drug in a constant ratio.

Brief Description of Drawings

[Figure 1] Figure 1 is a diagram showing the amino acid sequence of a heavy chain of an anti-TROP2 antibody (SEQ ID NO: 1).

[Figure 2] Figure 2 is a diagram showing the amino acid sequence of a light chain of an anti-TROP2 antibody (SEQ ID NO: 2).

[Figure 3] Figure 3 is a diagram showing the amino acid sequence of a heavy chain of an anti-B7-H3 antibody (SEQ ID NO: 3).

[Figure 4] Figure 4 is a diagram showing the amino acid sequence of a light chain of an anti-B7-H3 antibody (SEQ ID NO: 4).

[Figure 5] Figure 5 is a diagram showing the temporal variation of the content of D4 in D0 to D8 in production of an anti-TROP2 antibody-drug conjugate composition.

[Figure 6] Figure 6 is a diagram showing the temporal variations of the composition ratios of D4-1 and D4-2 in D4 in production of an anti-TROP2 antibody-drug conjugate composition.

[Figure 7] Figure 7 is a diagram showing the temporal variation of the content of D4 in D0 to D8 in production of an anti-B7-H3 antibody-drug conjugate composition.

[Figure 8] Figure 8 is a diagram showing the temporal variations of the composition ratios of D4-1 and D4-2 in D4 in production of an anti-B7-H3 antibody-drug conjugate composition.

Description of Embodiments

Hereinafter, preferred modes for carrying out the present invention are described. The embodiments described below are given merely for illustrating one example of a typical embodiment of the present invention and are not intended to limit the scope of the present invention.

1. Antibody-drug conjugate

In the present invention, an "antibody-drug conjugate" refers to a complex having a drug with cytotoxicity conjugated to an antibody via a linker. Examples of antibody-drug conjugates include those described in U.S. Patent

No. 6214345, International Publication No. 2002/083067, International Publication No. 2003/026577, International Publication No. 2004/054622, International Publication No. 2005/112919, International Publication No. 2006/135371, International Publication No. 2007112193, International Publication No. 2008/033891, International Publication No. 2009/100194, International Publication No. 2009/134976, International Publication No. 2009/134977, International Publication No. 2010/093395, International Publication No. 2011/130613, International Publication No. 2011/130616, International Publication No. 2013/055993, International Publication No. 2014/057687, International Publication No. 2014/107024, International Publication No. 2014/134457, International Publication No. 2014/145090, and International Publication No. 2015/098099. Preferably, those described in International Publication No. 2014/057687 and International Publication No. 2015/098099 can be exemplified.

The drug with cytotoxicity is not particularly limited as long as it has antitumor effect and has a substituent or partial structure capable of binding to a linker, and examples thereof include camptothecin, calicheamicin, doxorubicin, daunorubicin, mitomycin C, bleomycin, cyclocytidine, vincristine, vinblastine, methotrexate, cisplatin, auristatin E, maytansine, paclitaxel, pyrrolobenzodiazepine, and derivatives of them. Camptothecin derivatives can be preferably exemplified, and exatecan derivatives can be more preferably exemplified.

Exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinoline-10,13-dione (also represented by chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinoline-10,13(9H,15H)-dione)), which is a topoisomerase I inhibitor, is a compound represented by the formula:

[Formula 5]

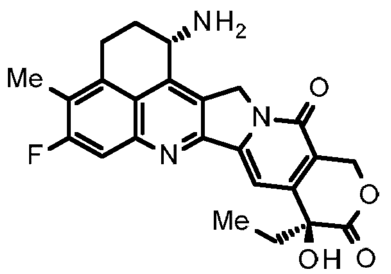

In the present invention, a "drug-linker" refers to drug and linker parts in an antibody-drug conjugate, in other words, a partial structure except an antibody in an antibody-drug conjugate.

In the present invention, an "interchain disulfide" refers to a disulfide between two heavy chains (heavy-heavy interchain disulfide) or a disulfide between a heavy chain and a light chain (heavy-light interchain disulfide) in an antibody.

In the present invention, an "interchain thiol" refers to a thiol group obtained by reduction of an interchain disulfide in an antibody.

In the present invention, a "heavy-heavy interchain thiol" refers to a thiol group obtained by reduction of a heavy-heavy interchain disulfide in an antibody.

In the present invention, a "heavy-light interchain thiol" refers to a thiol group obtained by reduction of a heavy-light interchain disulfide in an antibody.

The number of conjugated drug molecules per antibody molecule in an antibody-drug conjugate is an important factor that affects efficacy and safety. Four interchain disulfides are present in an antibody, and the number of conjugated drug molecules per antibody molecule is two, four, six, or eight since a disulfide is formed from two thiol groups.

As antibody-drug conjugates having two conjugated drug molecules per antibody molecule (hereinafter, also referred to as "D2"), two isomers, specifically, an antibody-drug conjugate having two units of a drug-linker conjugated to heavy-light interchain thiols (hereinafter, also referred to as "D2-1") and an antibody-drug conjugate having two units of a drug-linker conjugated to heavy-heavy interchain thiols (hereinafter, also referred to as "D2-2") can be formed:

[Formula 6]

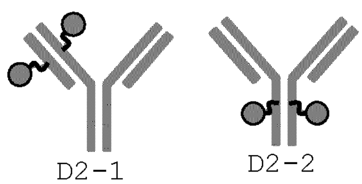

wherein each long bar (bent line) represents a heavy chain, each short bar represents a light chain, and each object consisting of a circle and a wavy line represents a unit of a drug-linker.

As antibody-drug conjugates having four conjugated drug molecules per antibody molecule (hereinafter, also referred to as "D4"), three isomers, specifically, an antibody-drug conjugate having four units of a drug-linker conjugated to heavy-light interchain thiols (hereinafter, also referred to as "D4-1"), an antibody-drug conjugate having four units of a drug-linker conjugated to heavy-heavy interchain thiols (hereinafter, also referred to as "D4-2"), and an antibody-drug conjugate having two units of a drug-linker conjugated to heavy-light interchain thiols and two units of a drug-linker conjugated to heavy-heavy interchain thiols (hereinafter, also referred to as "D4-3") can be formed:

[Formula 7]

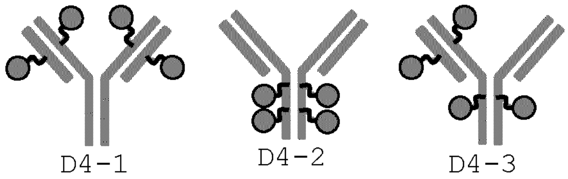


wherein each long bar (bent line) represents a heavy chain, each short bar represents a light chain, and each object consisting of a circle and a wavy line represents a unit of a drug-linker.

As antibody-drug conjugates having six conjugated drug molecules per antibody molecule (hereinafter, also referred to as "D6"), two isomers, specifically, an antibody-drug conjugate having four units of a drug-linker conjugated to heavy-light interchain thiols and two units of a drug-linker conjugated to heavy-heavy interchain thiols (hereinafter, also referred to as "D6-1") and an antibody-drug conjugate having two units of a drug-linker conjugated to heavy-light interchain thiols and four units of a drug-linker conjugated to heavy-heavy interchain thiols (hereinafter, also referred to as "D6-2") can be formed:

[Formula 8]

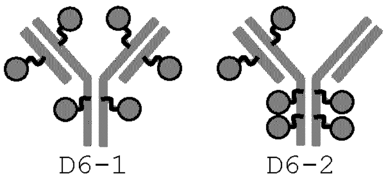


wherein each long bar (bent line) represents a heavy chain, each short bar represents a light chain, and each object consisting of a circle and a wavy line represents a unit of a drug-linker.

As an antibody-drug conjugate having eight conjugated drug molecules per antibody molecule (hereinafter, also referred to as "D8"), an antibody-drug conjugate having four units of a drug-linker conjugated to heavy-light interchain thiols and four units of a drug-linker conjugated to heavy-heavy interchain thiols can be formed:

[Formula 9]

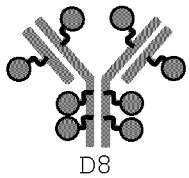


wherein each long bar (bent line) represents a heavy chain, each short bar represents a light chain, and each object consisting of a circle and a wavy line represents a unit of a drug-linker.

For example, each interchain thiol in an antibody binds to position 3 of the N-substituted maleimidyl group of a drug-linker intermediate to form thioether. That is, the binding part between an antibody and a drug-linker is represented, for example, by the following formula:

[Formula 10]

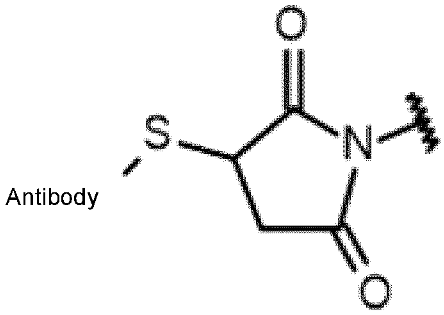

wherein "Antibody-S-" is derived from the antibody.

In the present invention, an "antibody-drug conjugate composition" refers to a composition that can contain D2, D4, D6, D8, and an antibody having no unit of a drug-linker conjugated (hereinafter, also referred to as "D0") at arbitrary proportions.

In the present invention, the "average number of conjugated drug molecules" is also called the drug-to-antibody ratio (DAR), and refers to the average number of drug molecules (or units of a drug-linker) that are conjugating per antibody molecule in an antibody-drug conjugate composition.

In the present invention, "contents" of isomers in an antibody-drug conjugate composition are expressed in mol% based on the antibody.

An antibody-drug conjugate preferably produced in the present invention is an antibody-drug conjugate in which a drug-linker represented by the formula:

[Formula 11]

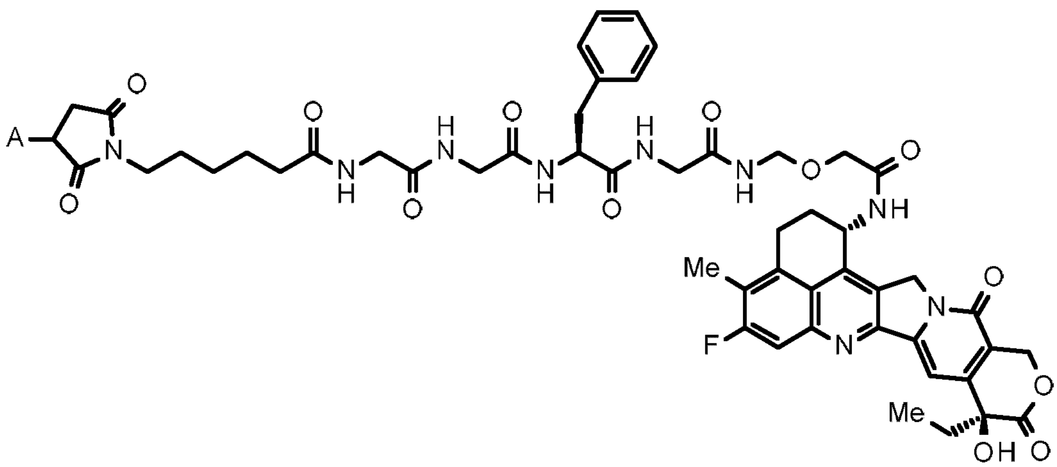

wherein A represents the connecting position to the antibody,
is conjugated to the antibody via a thioether bond. This drug-linker is connected to a thiol group (in other words, the sulfur atom of a cysteine residue) formed at an interchain disulfide bond site (two sites between heavy chains, and two sites between a heavy chain and a light chain) in the antibody.

The antibody-drug conjugate preferably produced in the present invention can also be represented by the following formula:

[Formula 12]

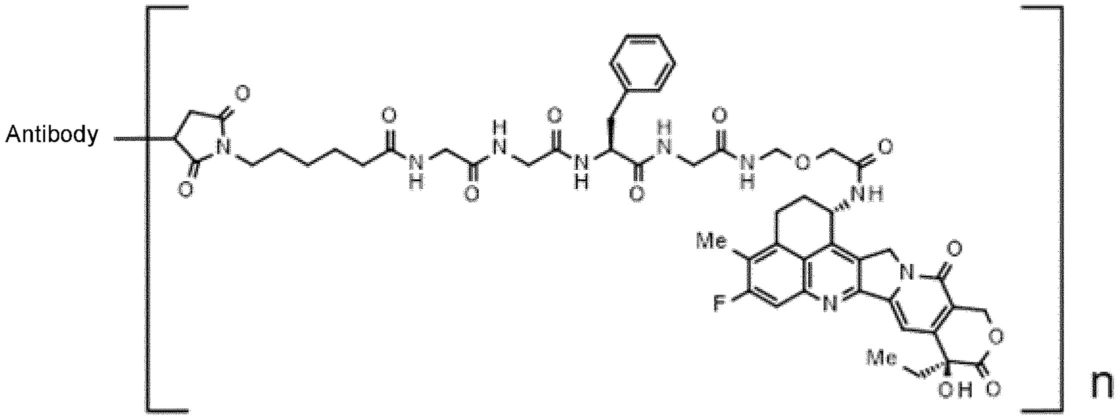

wherein, the drug-linker is conjugated to the antibody via a thioether bond. The meaning of n is the same as that of what is called the average number of conjugated drug molecules (DAR: drug-to-antibody ratio), and indicates the average number of units of the drug-linker conjugated per antibody molecule.

After migrating into cancer cells, the antibody-drug conjugate preferably produced in the present invention releases a compound represented by the formula:

[Formula 13]

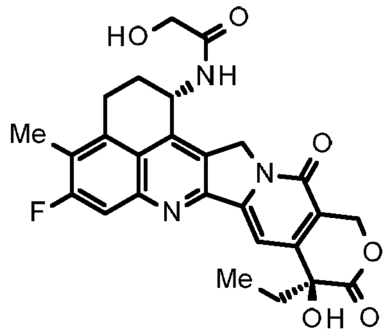

to exert an antitumor effect.

This compound is inferred to be the main source of the antitumor activity of the antibody-drug conjugate preferably produced in the present invention (International Publication No. 2014/057687 and International Publication No. 2015/098099).

This compound is inferred to be formed by decomposition of the aminal structure of a compound represented by the formula:

[Formula 14]

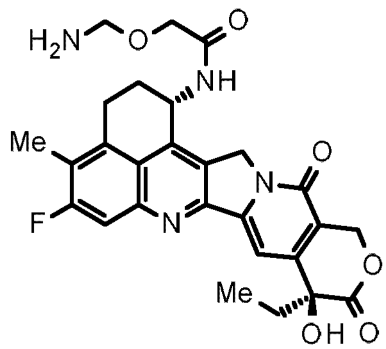

which is inferred to be formed by cleavage at the linker part of the antibody-drug conjugate.

2. Antibody for use in production of antibody-drug conjugate composition

The antibody for use in production of the antibody-drug conjugate composition of the present invention may be derived from any species, and is preferably an antibody derived from a human, a rat, a mouse, or a rabbit. In cases when the antibody is derived from species other than human species, it is preferably chimerized or humanized using a well-known technique. The antibody of the present invention may be a polyclonal antibody or a monoclonal antibody and is preferably a monoclonal antibody.

The antibody for use in production of the antibody-drug conjugate composition of the present invention is an antibody preferably having a characteristic of being capable of targeting cancer cells, and is preferably an antibody possessing, for example, a property of recognizing a cancer cell, a property of binding to a cancer cell, a property of being incorporated and internalized in a cancer cell, and/or cytocidal activity against cancer cells.

The binding activity of the antibody against cancer cells can be confirmed using flow cytometry. The internalization of the antibody into cancer cells can be confirmed, for example, using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed in vivo, for example, by administering the antibody to a nude mouse with a transplanted cancer cell line highly expressing the target protein, and determining change in the cancer cell.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of migrating into (being internalized in) cancer cells.

The antibody for use in production of the antibody-drug conjugate composition of the present invention can be obtained by a procedure known in the art. For example, the antibody of the present invention can be obtained using a method conventionally carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen are fused with myeloma cells according to a method known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The antibody for use in production of the antibody-drug conjugate composition of the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)).

As the humanized antibody, an antibody obtained by integrating only the complementarity determining region (CDR) of a heterologous antibody into a human-derived antibody (Nature (1986) 321, p. 522-525), and an antibody

obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology:Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002)43 (7), p.2301-2308; Carmen, S. et. al., Briefings in Functional Genomics and Proteomics (2002), 1(2), p.189-203; Siriwardena, D. et. al., Ophthalmology (2002) 109(3), p.427-431, etc.) can be exemplified.

Modified variants of the antibody for use in production of the antibody-drug conjugate composition of the present invention are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, amino- or carboxyl-terminal processing, deamidation, isomerization of aspartic acid, or oxidation of methionine), and variants in which a methionine residue has been added to the amino terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, WO 02/31140, etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions. However, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified, and IgG1 or IgG2 can be preferably exemplified.

Examples of antibodies applicable to production of the antibody-drug conjugate composition of the present invention can include, but are not particularly limited to, an anti-TROP2 antibody, an anti-B7-H3 antibody, an anti-HER2 antibody, an anti-HER3 antibody, an anti-CD3 antibody, an anti-CD30 antibody, an anti-CD33 antibody, an anti-CD37 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA antibody, an anti-Mesothelin antibody, an anti-A33 antibody, an anti-CanAg antibody, an anti-Cripto antibody, an anti-G250 antibody, an anti-MUC1 antibody, an anti-GPNMB antibody, an anti-Integrin antibody, an anti-Tenascin-C antibody, an anti-SLC44A4

antibody, an anti-GPR20 antibody, and an anti-CDH6 antibody. Further, an anti-TROP2 antibody and an anti-B7-H3 antibody can be preferably exemplified.

In the present invention, the term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.

Examples of the anti-TROP2 antibody can include an antibody described in International Publication No. 2015/098099,

preferably an antibody comprising a heavy chain comprising CDRH1 consisting of an amino acid sequence represented by SEQ ID NO: 5 (an amino acid sequence consisting of amino acid residues 50 to 54 of SEQ ID NO: 1), CDRH2 consisting of an amino acid sequence represented by SEQ ID NO: 6 (an amino acid sequence consisting of amino acid residues 69 to 85 of SEQ ID NO: 1) and CDRH3 consisting of an amino acid sequence represented by SEQ ID NO: 7 (an amino acid sequence consisting of amino acid residues 118 to 129 of SEQ ID NO: 1), and a light chain comprising CDRL1 consisting of an amino acid sequence represented by SEQ ID NO: 8 (an amino acid sequence consisting of amino acid residues 44 to 54 of SEQ ID NO: 2), CDRL2 consisting of an amino acid sequence represented by SEQ ID NO: 9 (an amino acid sequence consisting of amino acid residues 70 to 76 of SEQ ID NO: 2) and CDRL3 consisting of an amino acid sequence represented by SEQ ID NO: 10 (an amino acid sequence consisting of amino acid residues 109 to 117 of SEQ ID NO: 2),

more preferably an antibody comprising a heavy chain comprising a heavy chain variable region consisting of an amino acid sequence consisting of amino acid residues 20 to 140 of SEQ ID NO: 1 and a light chain comprising a light chain variable region consisting of an amino acid sequence consisting of amino acid residues 21 to 129 of SEQ ID NO: 2,

and even more preferably an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted.

In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.

Examples of the anti-B7-H3 antibody can include M30-H1-L4 (International Publication No. 2014/057687),

preferably an antibody comprising a heavy chain comprising CDRH1 consisting of an amino acid sequence represented by SEQ ID NO: 11 (an amino acid sequence consisting of amino acid residues 50 to 54 of SEQ ID NO: 3), CDRH2 consisting of an amino acid sequence represented by SEQ ID NO: 12 (an amino acid sequence consisting of amino acid residues 69 to 85 of SEQ ID NO: 3) and CDRH3 consisting of an amino acid sequence represented by SEQ ID NO: 13 (an amino acid sequence consisting of amino acid residues 118 to 130 of SEQ ID NO: 3), and a light chain comprising CDRL1 consisting of an amino acid sequence represented by SEQ ID NO: 14 (an amino acid sequence consisting of amino acid residues 44 to 53 of SEQ ID NO: 4), CDRL2 consisting of an amino acid sequence represented by SEQ ID NO: 15 (an amino acid sequence consisting of amino acid residues 69 to 75 of SEQ ID NO: 4) and CDRL3 consisting of an amino acid sequence represented by SEQ ID NO: 16 (an amino acid sequence consisting of amino acid residues 108 to 116 of SEQ ID NO: 4),

more preferably an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4,

and even more preferably an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted.

In the present invention, an "anti-TROP2 antibody-drug conjugate composition" refers to such an antibody-drug conjugate composition that the antibody in the antibody-drug conjugate composition produced in the present invention is an anti-TROP2 antibody.

In the present invention, an "anti-B7-H3 antibody-drug conjugate composition" refers to such an antibody-drug conjugate composition that the antibody in the antibody-drug conjugate composition produced in the present invention is an anti-B7-H3 antibody.

3. Drug-linker intermediate for use in production of antibody-drug conjugate composition

A drug-linker intermediate for use in production of the antibody-drug conjugate composition of the present invention is not particularly limited as long as it is a compound subjected to reaction with interchain thiols of an antibody, preferably a compound having an N-substituted maleimidyl group, and more preferably a compound represented by the following formula:

[Formula 15]

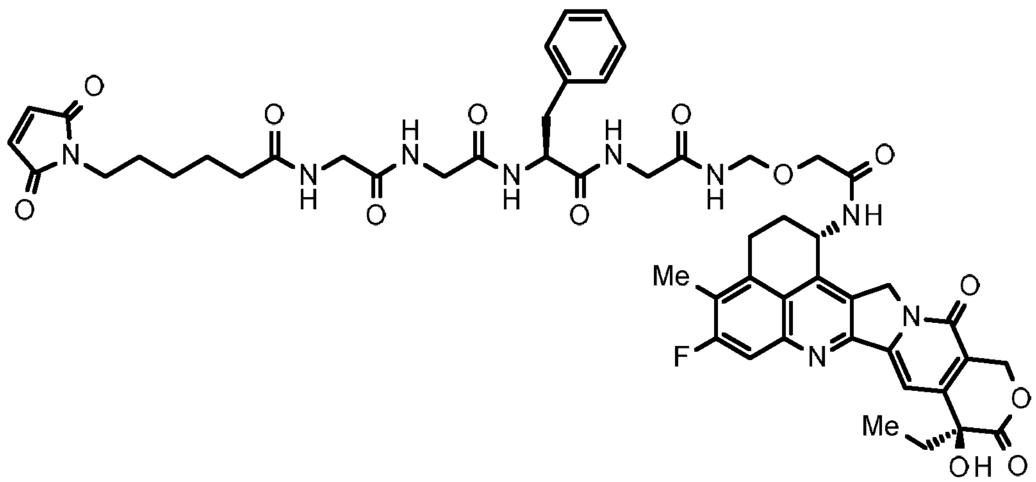

This drug-linker intermediate can be represented by the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. 2014/057687, International Publication No. 2015/098099, International Publication No. 2019/044947, and so on.

4. Conjugation of antibody and drug-linker intermediate

In the present invention, conjugation of an antibody and a drug-linker intermediate includes:

(i) a step of reacting an antibody with a reducing agent to obtain an antibody having thiol groups; then
(ii) a step of reacting drug-linker intermediates with the antibody having thiol groups obtained in the step (i), wherein

the step (i) is carried out until the composition ratio of the antibody having four heavy-light interchain thiols and the composition ratio of the antibody having four heavy-heavy interchain thiols reach a steady value (equilibration of reduction reaction). Thereby, an antibody-drug conjugate composition comprising each of the isomers of the antibody-drug conjugate having different binding positions of the drug in a constant ratio can be stably produced.

In the present invention, "reach a steady value" means that the corresponding composition ratio (%) comes to a constant value over time (it may vary within a range substantially recognized as a constant value, may vary preferably within a range of $\pm$5%, more preferably within a range of $\pm$4%, even more preferably within a range of $\pm$3%, even more preferably within a range of $\pm$2%, even more preferably within a range of $\pm$1%).

The composition ratio of the antibody having four heavy-light interchain thiols at each reaction time can be assumed to be identical to the composition ratio of D4-1 in the antibody-drug conjugate composition produced in the reaction time (content of D4-1/(content of D4-1 + content of D4-2) $\times$ 100).

The composition ratio of the antibody having four heavy-heavy interchain thiols at each reaction time can be assumed to be identical to the composition ratio of D4-2 in the antibody-drug conjugate composition produced in the reaction time (content of D4-2/(content of D4-1 + content of D4-2) $\times$ 100).

Accordingly, the time at which the composition ratio of the antibody having four heavy-light interchain thiols and the composition ratio of the antibody having four heavy-heavy interchain thiols reach a steady value can be identified by tracing the temporal variation of each of the composition ratios of D4-1 and D4-2 in the antibody-drug conjugate composition produced and confirming the time at which each of these reaches a constant value (it may vary within a

range substantially recognized as a constant value, preferably within a range of ±5%, more preferably within a range of ±4%, even more preferably within a range of ±3%, even more preferably within a range of ±2%, even more preferably within a range of ±1%).

The step (i) can be preferably carried out for 2 hours or more, more preferably carried out for 4 hours or more, 12 hours or more, 16 hours or more, 20 hours or more, or 31 hours or more, even more preferably carried out for 5 hours or more, 12 hours or more, 20 hours or more, 24 hours or more, or 48 hours or more, and even more preferably carried out for 6 hours or more, 12 hours or more, 20 hours or more, 24 hours or more (or 36 hours or more), or 48 hours or more, and in the form including an upper limit value, the step (i) can be preferably carried out for 2 hours to 168 hours, more preferably carried out for 4 hours to 50 hours, 12 hours to 48 hours, 16 hours to 48 hour, 20 hours to 51 hours, or 31 hours to 50 hours, even more preferably carried out for 5 hours to 50 hours, 12 hours to 48 hours, 20 hours to 51 hours, 24 hours to 48 hours, or 48 hours to 50 hours, and even more preferably carried out for 6 hours to 50 hours, 12 hours to 48 hours, 20 hours to 51 hours, 24 hours to 48 hours (or 36 hours to 48 hours), or 48 hours to 50 hours.

The step (i) can be preferably carried out at -5°C to 35°C, more preferably carried out at 0 to 20°C, even more preferably carried out at 5 to 20°C, and even more preferably carried out at 5 to 10°C, or about 10°C. In the present invention, "about 10°C" is preferably 8°C to 12°C, more preferably 9°C to 11°C, and even more preferably 10°C to 11°C, or 10°C.

In the case that the step (i) is carried out at 20°C, the step (i) can be preferably carried out for 4 hours or more, more preferably carried out for 5 hours or more, and even more preferably carried out for 6 hours or more, and in the form including an upper limit value, the step (i) can be preferably carried out for 4 hours to 50 hours, more preferably carried out for 5 hours to 50 hours, and even more preferably carried out for 6 hours to 50 hours.

In the case that the step (i) is carried out at 10°C, the step (i) can be preferably carried out for 12 hours or more or 20 hours or more, and in the form including an upper limit value, the step (i) can be preferably carried out for 12 hours to 48 hours or 20 hours to 51 hours.

In the case that the step (i) is carried out at 5°C, the step (i) can be preferably carried out for 16 hours or more, more preferably carried out for 20 hours or more, and even more preferably carried out for 24 hours or more (or 36 hours or more), and in the form including an upper limit value, the step (i) can be preferably carried out for 16 hours to 48 hours, more preferably carried out for 20 hours to 48 hours, and even more preferably carried out for 24 hours to 48 hours (or 36 hours to 48 hours).

In the case that the step (i) is carried out at 0°C, the step (i) can be preferably carried out for 31 hours or more, and more preferably carried out for 48 hours or more, and in the form including an upper limit value, the step (i) can be preferably carried out for 31 hours to 50 hours, and more preferably carried out for 48 hours to 50 hours.

The reaction time and reaction temperature in the step (i) can be further optimized in accordance with the type of the antibody in the antibody-drug conjugate composition to be produced.

Specifically, in the case that the antibody in the antibody-drug conjugate composition to be produced is an anti-TROP2 antibody (preferably, an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted), the step (i) can be preferably carried out at about 10°C for 20 hours or more, and in the form including an upper limit value, the step (i) can be preferably carried out at about 10°C for 20 hours to 51 hours.

In the case that the antibody in the antibody-drug conjugate composition to be produced is an anti-B7-H3 antibody (preferably, an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted), the step (i) can be preferably carried out at 5 to 10°C (more preferably 6°C, 7°C, 8°C, 9°C, or 10°C) for 12 hours or more, or at 5°C for 20 hours or more, and in the form including an upper limit value, the step (i) can be preferably carried out at 5 to 10°C (more preferably 6°C, 7°C, 8°C, 9°C, or 10°C) for 12 hours to 48 hours, or at 5°C for 20 hours to 48 hours.

The reducing agent used in the step (i) is not particularly limited as long as it is capable of reducing an interchain disulfide of the antibody, and, for example, tris(2-carboxyethyl)phosphine or a salt thereof, dithiothreitol, or 2-mercaptoethanol can be used, tris(2-carboxyethyl)phosphine or a salt thereof can be preferably used, and tris(2-carboxyethyl)phosphine hydrochloride can be more preferably used.

The equivalent (hereinafter, an "equivalent" refers to a molar equivalent in the present invention) of the reducing agent used in step (i) per antibody molecule is preferably 1.9 to 2.5 equivalents per antibody molecule, and more preferably 2 to 2.2 equivalents per antibody molecule.

The solvent used in the step (i) is not particularly limited as long as it allows the reaction to progress, and an aqueous solution of L-histidine can be preferably used.

The solvent used in the step (i) can be further optimized in accordance with the type of the antibody in the

antibody-drug conjugate composition to be produced.

Specifically, in the case that the antibody in the antibody-drug conjugate composition to be produced is an anti-TROP2 antibody (preferably, an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted), the solvent used in the step (i) is preferably 0.001 mol/L to 0.1 mol/L aqueous solution of L-histidine, more preferably 0.005 mol/L to 0.02 mol/L aqueous solution of L-histidine, and even more preferably 0.01 mol/L aqueous solution of L-histidine. The pH of the reaction solution in the step (i) can be preferably adjusted by using an aqueous solution of disodium hydrogen phosphate, and the pH is preferably 6.4 to 7.4, more preferably 6.7 to 7.1, and even more preferably 6.9.

In the case that the antibody in the antibody-drug conjugate composition to be produced is an anti-B7-H3 antibody (preferably, an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted), the solvent used in the step (i) is preferably 0.005 mol/L to 0.3 mol/L aqueous solution of L-histidine, more preferably 0.025 mol/L to 0.1 mol/L aqueous solution of L-histidine, and even more preferably 0.05 mol/L aqueous solution of L-histidine.

The reaction solution in the step (i) may contain a buffer solution derived from antibody production.

The step (i) is preferably performed in the presence of a chelating agent. The chelating agent is not particularly limited as long as it can be used in reducing an interchain disulfide of the antibody, and, for example, ethylenediaminetetraacetic acid (hereinafter, also referred to as "EDTA"), diethylenetriaminepentaacetic acid, or glycol ether diaminetetraacetic acid can be used, and ethylenediaminetetraacetic acid can be preferably used.

Preferably, 1 to 20 equivalents of the chelating agent can be used per antibody molecule, 3 to 8 equivalents of the chelating agent can be more preferably used per antibody molecule, 4 to 6 equivalents of the chelating agent can be even more preferably used per antibody molecule, and 5 or 6 equivalents of the chelating agent can be even more preferably used per antibody molecule.

The buffer solution used in step (i) may contain a surfactant. The term "surfactant" in the present invention refers to a substance which has a hydrophilic group and a hydrophobic group and can be used as one of the components of a pharmaceutical preparation. Examples of such surfactants include polysorbates (including polysorbate 80 (Tween 80), polysorbate 20 (Tween 20), and polysorbate 60 (Tween 60)), polyoxyethylene (160) polyoxypropylene (30) glycol, polyoxyethylene hydrogenated castor oil 60, polyoxyethylene castor oil, and sodium laurylsulfate, and polysorbate 20 and polysorbate 80 can be more preferably exemplified.

The type of the surfactant used in the step (i) can be appropriately selected in accordance with the type of the antibody in the antibody-drug conjugate composition to be produced.

In the case that the antibody in the antibody-drug conjugate composition to be produced is an anti-TROP2 antibody (preferably, an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted), for example, the buffer solution used in the step (i) contains polysorbate 80, and can be preferably used.

In the case that the antibody in the antibody-drug conjugate composition to be produced is an anti-B7-H3 antibody (preferably, an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted), the buffer solution used in the step (i) contains polysorbate 20, and can be preferably used.

Preferably, in the step (ii), 4 to 5 equivalents of the drug-linker intermediate can be used per antibody molecule, 4.2 to 4.6 equivalents of the drug-linker intermediate can be more preferably used per antibody molecule, and 4.4 equivalents of the drug-linker intermediate can be even more preferably used per antibody molecule.

The drug-linker intermediate used in the step (ii) in a state dissolved in a solvent can be preferably added to the reaction solution obtained in step (i). The solvent is not particularly limited as long as it can be used in the binding reaction with the antibody, and dimethylsulfoxide, an aqueous solution of dimethylsulfoxide, acetone, or an aqueous solution of acetone can be preferably used, an aqueous solution of dimethylsulfoxide can be more preferably used, and an 80% aqueous solution of dimethylsulfoxide can be even more preferably used. Further, any of these solvents can be preferably used with acetic acid contained therein, and can be more preferably used with an aqueous solution of acetic acid contained therein.

The step (ii) can be preferably carried out at 0 to 20°C, more preferably carried out at 5 to 20°C, and even more preferably carried out at 5 to 10°C, or about 10°C.

The reaction time for step (ii) is preferably 20 minutes to 4 hours, and more preferably 0.5 to 2 hours.

After the step (ii), a reagent having a thiol group can be used to quench an excess portion of the drug-linker intermediate.

The reagent having a thiol group is not particularly limited as long as it can react with the maleimidyl group of the drug-linker intermediate, and, for example, N-acetylcysteine or cysteine can be used, and N-acetylcysteine can be preferably used.

Preferably, 2 to 8 equivalents of the reagent having a thiol group can be used, 3 to 7 equivalents of the reagent having a thiol group can be more preferably used, and 3 to 5 equivalents of the reagent having a thiol group can be even more preferably used.

The step of adding the reagent having a thiol group can be preferably carried out at 0 to 20°C, more preferably carried out at 5 to 20°C, and even more preferably carried out at 5 to 10°C, or about 10°C.

The antibody-drug conjugate composition obtained can be purified by removing impurities derived from the drug-linker intermediate through ultrafiltration.

The term "ultrafiltration" in the present invention refers to a purification method to separate large solute molecules and small solute molecules or separate solute molecules and solvent molecules by filtration through a membrane (ultrafiltration membrane) having a pore size of from about 0.001 μm to about 0.05 μm. In general, ultrafiltration membranes have a molecular weight cutoff (MWCO) in the range of from 1 kDa to 1000 kDa. MWCO is generally defined as the molecular weight of a spherical solute such that 90% of the spherical solute molecules are retained by the membrane. The ultrafiltration in the present invention can be preferably performed by using an ultrafiltration membrane with MWCO of 1 kDa to 100 kDa, and more preferably performed by using an ultrafiltration membrane with MWCO of 30 kDa. Examples of the material of the ultrafiltration membrane include regenerated cellulose, cellulose acetate, aromatic polyamide, polyvinyl alcohol, polysulfone, polyether sulfone, polyvinylidene fluoride, polyethylene, polyacrylonitrile, nylon, and ceramics. The ultrafiltration in the present invention can be preferably performed by using an ultrafiltration membrane the material of which is regenerated cellulose, though the material is not limited thereto. Examples of the ultrafiltration membrane used in the present invention can include a Pellicon (R) XL Cassette Ultracel (R) (produced by Merck KGaA), a Pellicon (R) 2 Ultracel (R) (produced by Merck KGaA), and a Pellicon (R) 3 Ultracel (R) (produced by Merck KGaA). Ultrafiltration particularly refers to a method of forcibly filtering through pressure control or centrifugation. On the other hand, methods of filtering through passive diffusion may be generally referred to as "diafiltration". However, any of the methods using an ultrafiltration membrane is included in the scope of "ultrafiltration" in the present invention.

The average number of conjugated drug molecules of the antibody-drug conjugate composition produced in the present invention is preferably 2 to 6, more preferably 3 to 5, even more preferably 3.5 to 4.5, and even more preferably about 4. In the present invention, "about 4" is preferably 3.8 to 4.2, more preferably 3.9 to 4.1, and even more preferably 4.

The content of the antibody-drug conjugate having four units of a drug-linker conjugated (D4) in the antibody-drug conjugate composition produced in the present invention is preferably 50% or more, and in the form including an upper limit value, preferably 50 to 59%, 50 to 60%, 50 to 70%, 50 to 80%, 50 to 90%, or 50 to 100%.

Further, the composition ratio of the antibody-drug conjugate having four units of a drug-linker conjugated to heavy-light interchain thiols in the antibody-drug conjugate composition produced in the present invention is preferably 1.5 to 2.5 times, more preferably 1.5 to 2.33 times the composition ratio of the antibody-drug conjugate having four units of a drug-linker conjugated to heavy-heavy interchain thiols.

In other words, the ratio between the content of the antibody-drug conjugate having four units of a drug-linker conjugated to heavy-light interchain thiols and the content of the antibody-drug conjugate having four units of a drug-linker conjugated to heavy-heavy interchain thiols is preferably 60:40 to 71:29, and more preferably 60:40 to 70:30 in the antibody-drug conjugate composition produced in the present invention.

5. Evaluation of properties of antibody-drug conjugate composition

The average number of conjugated drug molecules per antibody molecule of the antibody-drug conjugate composition produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method) (see International Publication No. 2014/057687, International Publication No. 2015/098099, International Publication No. 2017/002776, etc.). The measurement conditions in the HPLC method can be optimized in accordance with the type of the antibody in the antibody-drug conjugate composition to be produced.

(1-1) Preparation of sample for HPLC analysis (reduction of antibody-drug conjugate)

A diluted solution of an antibody-drug conjugate composition (60 μL) is mixed with an aqueous solution of

dithiothreitol (DTT) (100 mM, 15 μL). A sample in which the interchain disulfide bond of the antibody-drug conjugate has been cleaved by incubating the mixture for 20 minutes or 30 minutes at 37°C is used in HPLC analysis.

(1-2) HPLC analysis

In the case that the antibody in the antibody-drug conjugate composition to be produced is an anti-TROP2 antibody (preferably, an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted), for example, HPLC analysis can be performed as follows.

Measurement apparatus: high-performance liquid chromatograph
Detector: ultraviolet absorption spectrometer (measurement wavelength: 280 nm)
Column: PLRP-S (2.1 × 50 mm, 8 μm, 1000 angstroms; Agilent Technologies, Inc.)
Column temperature: 80°C
Mobile phase A: aqueous solution containing 0.05% trifluoroacetic acid (TFA)
Mobile phase B: acetonitrile solution containing 0.04% TFA
Gradient program (mobile phase B): 27%-36% (0-12.5 min), 36%-42% (12.5-15 min), 42%-27% (15-15.1 min), 27%-27% (15.1-25 min)

In the case that the antibody in the antibody-drug conjugate composition to be produced is an anti-B7-H3 antibody (preferably, an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted), HPLC analysis can be performed as follows.

Measurement apparatus: high-performance liquid chromatograph
Detector: ultraviolet absorption spectrometer (measurement wavelength: 280 nm)
Column: PLRP-S (2.1 × 50 mm, 8 μm, 1000 angstroms; Agilent Technologies, Inc.)
Column temperature: 80°C
Mobile phase A: aqueous solution containing 0.05% trifluoroacetic acid (TFA)
Mobile phase B: acetonitrile solution containing 0.04% TFA
Gradient program (mobile phase B): 29%-36% (0-12.5 min), 36%-42% (12.5-15 min), 42%-29% (15-15.1 min), 29%-29% (15.1-25 min)

(1-3) Data analysis

Compared with non-conjugated antibody light ($L_0$) and heavy ($H_0$) chains, drug-conjugated light (light chain connected to one drug molecule: $L_1$) and heavy (heavy chain connected to one drug molecule: $H_1$, heavy chain connected to two drug molecules: $H_2$, heavy chain connected to three drug molecules: $H_3$) chains exhibit higher hydrophobicity in proportion to the number of conjugated drug molecules and thus have a larger retention time. These chains are therefore eluted in the order of $L_0$ and $L_1$ or $H_0$, $H_1$, $H_2$, and $H_3$. Detection peaks can be assigned to any of $L_0$, $L_1$, $H_0$, $H_1$, $H_2$, and $H_3$ by the comparison of retention times with $L_0$ and $H_0$.

Since the drug-linker has UV absorption, peak area values are corrected in response to the number of conjugated drug-linker molecules according to the following expression using the molar absorption coefficients of the light or heavy chain and the drug-linker.

[Math. 1]

Corrected value of the peak area of the light chain ($A_{Li}$)

$$= \text{Peak area} \times \frac{\text{Molar absorption coefficient of the light chain}}{\text{Molar absorption coefficient of the light chain} + \text{The number of conjugated drug molecules} \times \text{Molar absorption coefficient of the drug} - \text{linker}}$$

[Math. 2]

Corrected value of the peak area of the heavy chain ($A_{Hi}$)

$$= \text{Peak area} \times \frac{\text{Molar absorption coefficient of the heavy chain}}{\text{Molar absorption coefficient of the heavy chain} + \text{The number of conjugated drug molecules} \times \text{Molar absorption coefficient of the drug} - \text{linker}}$$

Here, as the molar absorption coefficient (280 nm) of the light or heavy chain of each antibody, a value estimated from the amino acid sequence of the light or heavy chain of each antibody by a known calculation method (Protein Science, 1995, vol. 4, 2411-2423) can be used.

In the case that the antibody is an anti-TROP2 antibody (an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2), for example, a molar absorption coefficient of 27640 and a molar absorption coefficient of 83810 can be used as estimated values for the light and heavy chains, respectively.

In the case that the antibody is an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4), a molar absorption coefficient of 30160 and a molar absorption coefficient of 87250 can be used as estimated values for the light and heavy chains, respectively.

As the molar absorption coefficient (280 nm) of the drug-linker, the measured molar absorption coefficient (280 nm) of a compound in which the maleimidyl group is converted to succinimide thioether by the reaction of each drug-linker intermediate with mercaptoethanol or N-acetylcysteine can be used.

The peak area ratio (%) of each chain is calculated for the total of the corrected values of peak areas according to the following expression.

[Math. 3]

$$\text{Peak area ratio of the light chain} = \frac{A_{Li}}{A_{L0}+A_{L1}} \times 100$$

$$\text{Peak area ratio of the heavy chain} = \frac{A_{Hi}}{A_{H0}+A_{H1}+A_{H2}+A_{H3}} \times 100$$

$A_{Li}$, $A_{Hi}$: Corrected values of respective peak areas of $L_i$, $H_i$

The average number of conjugated drug molecules in the antibody-drug conjugate composition is calculated according to the following expression.

Average number of conjugated drug molecules = ($L_0$ peak area ratio $\times$ 0 + $L_1$ peak area ratio $\times$ 1 + $H_0$ peak area ratio $\times$ 0 + $H_1$ peak area ratio $\times$ 1 + $H_2$ peak area ratio $\times$ 2 + $H_3$ peak area ratio $\times$ 3) / 100 $\times$ 2

Calculation of the number of conjugated drug molecules and contents of isomers having different binding positions of a drug in the antibody-drug conjugate composition produced can be performed, for example, with a method in which elution times for isomers are separated by using HPLC and area values of contents of isomers are measured (see International Publication No. 2017/002776, etc.). The HPLC method can be optimized in accordance with the type of the antibody in the antibody-drug conjugate composition to be produced.

(2-1) HPLC analysis

A diluted solution of an antibody-drug conjugate composition is used for HPLC analysis. Alternatively, a sample obtained by incubating the diluted solution at 37°C for 60 minutes is used for HPLC analysis.

In the case that the antibody in the antibody-drug conjugate composition to be produced is an anti-TROP2 antibody (preferably, an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2, or a variant of the antibody in which a lysine residue at the carboxyl terminus of the heavy chain is deleted), for example, HPLC analysis can be performed as follows.

Measurement apparatus: high-performance liquid chromatograph
Detector: ultraviolet absorption spectrometer (measurement wavelength: 280 nm)
Column: TSK-gel Butyl-NPR (4.6 × 35 mm, 2.5 μm; Tosoh Corporation)
Column temperature: 25°C
Mobile phase A: 30 mM phosphate buffer solution containing 1.5 M ammonium sulfate (pH 7.0)
Mobile phase B: mixed solution containing 90% of 30 mM phosphate buffer solution (pH 7.0) and 10% of isopropyl alcohol
Gradient program (mobile phase B): 25%-65% (0-15 min), 65%-95% (15-15.1 min), 95% (15.1-18 min), 95%-25% (18-18.1 min), 25% (18.1-25 min)

In the case that the antibody in the antibody-drug conjugate composition to be produced is an anti-B7-H3 antibody (preferably, an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted), for example, HPLC analysis can be performed as follows.

Measurement apparatus: high-performance liquid chromatograph
Detector: ultraviolet absorption spectrometer (measurement wavelength: 280 nm)
Column: TSK-gel Butyl-NPR (4.6 × 35 mm, 2.5 μm; Tosoh Corporation)
Column temperature: 25°C
Mobile phase A: 30 mM phosphate buffer solution containing 1.5 M ammonium sulfate (pH 7.0)
Mobile phase B: mixed solution containing 90% of 30 mM phosphate buffer solution (pH 7.0) and 10% of isopropyl alcohol
Gradient program (mobile phase B): 20%-64% (0-15 min), 64%-95% (15-15.1 min), 95% (15.1-18 min), 95%-20% (18-18.1 min), 20% (18.1-25 min)

(2-2) Data analysis

Under the HPLC separation conditions in (2-1), D0, D2, D4-1, D4-2, D6, and D8 are eluted in the order presented according to difference of their salt concentrations. Since the drug-linker has UV absorption, peak area values are corrected in response to the number of conjugated drug-linker molecules according to the following expression using the molar absorption coefficients of the antibody and drug-linker.

[Math. 4]

Corrected value of the peak area ($A_i$)

$$= \text{Peak area} \times \frac{\text{Molar absorption coefficient of the antibody}}{\text{Molar absorption coefficient of the antibody} + \text{The number of conjugated drug molecules} \times \text{Molar absorption coefficient of the drug} - \text{linker}}$$

Here, as the molar absorption coefficient (280 nm) of the light or heavy chain of each antibody, a value estimated from the amino acid sequence of the light or heavy chain of each antibody by a known calculation method (Protein Science, 1995, vol. 4, 2411-2423) can be used.

In the case that the antibody is an anti-TROP2 antibody (an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2), for example, a molar absorption coefficient of 223400 can be used as an estimated value for the antibody.

In the case that the antibody is an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4), a molar absorption coefficient of 235320 can be used as an estimated value for the antibody.

As the molar absorption coefficient (280 nm) of the drug-linker, the measured molar absorption coefficient (280 nm) of a compound in which the maleimidyl group is converted to succinimide thioether by the reaction of each drug-linker intermediate with mercaptoethanol or N-acetylcysteine can be used.

The peak area ratio (%) of each is calculated for the total of the corrected values of peak areas according to

the following expression, and thus the content of each isomer can be calculated.

[Math. 5]

$$\text{Peak area ratio} = \frac{A_i}{A_{D0} + A_{D2} + A_{D4-1} + A_{D4-2} + A_{D6} + A_{D8}} \times 100$$

$A_i$: Corrected values of respective peak areas

6. Pharmaceutical composition

The antibody-drug conjugate composition produced in the present invention can contain one or more pharmaceutically compatible components for administration. Pharmaceutically compatible components can be appropriately selected to apply from formulation additives and others commonly used in the field in accordance with the dosage of the antibody-drug conjugate composition produced in the present invention, the concentration thereof administered, and so on. For example, the antibody-drug conjugate composition produced in the present invention can be administered as a pharmaceutical composition containing a buffer such as histidine buffer, an excipient such as sucrose or trehalose, and a surfactant such as polysorbate 80 or polysorbate 20.

The pharmaceutical composition containing the antibody-drug conjugate composition produced in the present invention can be expected to exert a therapeutic effect by application as systemic therapy to patients, and additionally, by local application to cancer tissues.

The pharmaceutical composition containing the antibody-drug conjugate composition produced in the present invention can be preferably used for a mammal, but is more preferably used for a human.

The pharmaceutical composition containing the antibody-drug conjugate composition produced in the present invention can be preferably used as an injection, more preferably used as an aqueous injection or a lyophilized injection, and even more preferably used as a lyophilized injection.

In the case that the pharmaceutical composition containing the antibody-drug conjugate composition produced in the present invention is an aqueous injection, it can preferably be diluted with a suitable diluent and then given as an intravenous infusion. For the diluent, a dextrose solution (preferably, a 5% dextrose solution), physiological saline, and the like, can be exemplified.

In the case that the pharmaceutical composition containing the antibody-drug conjugate composition produced in the present invention is a lyophilized injection, it can preferably be dissolved in water for injection, subsequently a required amount can be diluted with a suitable diluent and then given as an intravenous infusion. For the diluent, a dextrose solution (preferably, a 5% dextrose solution), physiological saline, and the like, can be exemplified.

Examples of the administration route applicable to administration of the pharmaceutical composition containing the antibody-drug conjugate composition produced in the present invention can include intravenous, intradermal, subcutaneous, intramuscular, and intraperitoneal routes, and intravenous routes can be preferably exemplified.

The antibody-drug conjugate composition produced in the present invention can be administered to a human with intervals of 1 to 180 days, preferably administered once every one week, two weeks, three weeks, or four weeks, and even more preferably administered once every three weeks. The antibody-drug conjugate composition produced in the present invention can be administered at a dosage of about 0.001 to 100 mg/kg per administration, and preferably administered at a dosage of 0.8 to 12.4 mg/kg per administration. In the case that the antibody-drug conjugate composition used in the present invention is an anti-TROP2 antibody-drug conjugate composition, a dosage of 0.27 mg/kg, 0.5 mg/kg, 1.0 mg/kg, 2.0 mg/kg, 4.0 mg/kg, 6.0 mg/kg, or 8.0 mg/kg per administration can be preferably administered once every three weeks. In the case that the antibody-drug conjugate composition used in the present invention is an anti-B7-H3 antibody-drug conjugate composition, a dosage of 0.8 mg/kg, 1.6 mg/kg, 3.2 mg/kg, 4.8 mg/kg, 6.4 mg/kg, or 8.0 mg/kg per administration can be preferably administered once every three weeks.

The pharmaceutical composition containing the antibody-drug conjugate composition produced in the present invention can be used for treating cancer, and can be preferably used for treating at least one cancer selected from the group consisting of breast cancer (including triple-negative breast cancer, luminal breast cancer), gastric cancer (also called gastric adenocarcinoma), colorectal cancer (also called colon and rectal cancer and including colon cancer and rectal cancer), lung cancer (including small cell lung cancer and non-small cell lung cancer), esophageal cancer, head-and-neck cancer (including salivary gland cancer and pharyngeal cancer), esophagogastric junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulvar cancer, thyroid cancer, penis cancer, leukemia, malignant lymphoma, plasmacytoma, myeloma, glioblastoma multiforme,

sarcoma, osteosarcoma, and melanoma.

The pharmaceutical composition containing the antibody-drug conjugate composition produced in the present invention can be selectively used as an agent for drug therapy, which is a main method for treating cancer, and as a result can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of the cancer patients. Even if the pharmaceutical composition of the present invention does not accomplish killing cancer cells, it can achieve higher QOL of cancer patients while achieving longer-term survival, by inhibiting or controlling the growth of cancer cells.

In such drug therapy, the pharmaceutical composition containing the antibody-drug conjugate composition produced in the present invention can be used as an agent alone and, in addition, it can be used as an agent in combination with an additional therapy in adjuvant therapy and can be combined with surgical operation, radiotherapy, hormone therapy, or the like. Furthermore, it can also be used as an agent for drug therapy in neoadjuvant therapy.

In addition to the therapeutic use as described above, for example, a prophylactic effect such as suppressing the growth of small metastatic cancer cells and further killing them can also be expected for the pharmaceutical composition containing the antibody-drug conjugate composition produced in the present invention. For example, an effect of inhibiting and killing cancer cells in a body fluid in the course of metastasis or an effect of, for example, inhibiting and killing small cancer cells immediately after implantation in any tissue can be expected. Accordingly, inhibition of cancer metastasis or a prophylactic effect can be expected, particularly, after surgical removal of cancer.

The pharmaceutical composition containing the antibody-drug conjugate composition produced in the present invention can be administered in combination with other cancer treating agents. The antitumor effect may be enhanced accordingly. Examples of other cancer treating agents used for such purpose include irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, a tegafur/gimeracil/oteracil-containing agent, cetuximab, panitumumab, bevacizumab, ramucirumab, regorafenib, a trifluridine/tipiracil-containing agent, gefitinib, erlotinib, afatinib, osimertinib, methotrexate, pemetrexed, tamoxifen, toremifene, fulvestrant, leuprorelin, goserelin, letrozole, anastrozole, a progesterone formulation, trastuzumab emtansine, trastuzumab, pertuzumab, lapatinib, nivolumab, pembrolizumab, atezolizumab, durvalumab, avelumab, ipilimumab, tremelimumab, olaparib, rucaparib, niraparib, talazoparib, and veliparib, but such a cancer treating agent is not limited as long as it has antitumor activity.

Examples

The present invention is more specifically described with reference to the Examples shown below. However, the present invention is not limited to these.

[Example 1] Tracking of temporal variations of composition ratios of D4-1 and D4-2 in production of anti-TROP2 antibody-drug conjugate composition

A solution containing an anti-TROP2 antibody (an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2) (corresponding to 500 mg of the antibody) was placed in a polypropylene tube, to which 0.1 g/g aqueous solution of polysorbate 80 (50 μL), 0.5 mol/L aqueous solution of EDTA (5 equivalents to the antibody), and 0.01 mol/L aqueous solution of L-histidine (38 mL) were further added, and 0.3 mol/L aqueous solution of disodium hydrogen phosphate was then added thereto to adjust the pH to 6.9. Subsequently, an aqueous solution containing 1 mg/g tris(2-carboxyethyl)phosphine hydrochloride (2.12 g; 2.15 equivalents per antibody molecule) was added under stirring at 0°C, 10°C, or 20°C, and stirring was performed at the same temperature to form an antibody having thiol groups. After the lapse of 1 hour, 2 hours, 3 hours, 4 hours, 5 hours, 6 hours, 7 hours, 20 hours, 22 hours, 24 hours, 25 hours, 28 hours, 31 hours, 44 hours, 48 hours, 50 hours, or 51 hours, a part of the reaction solution was collected and a compound represented by the formula:

[Formula 16]

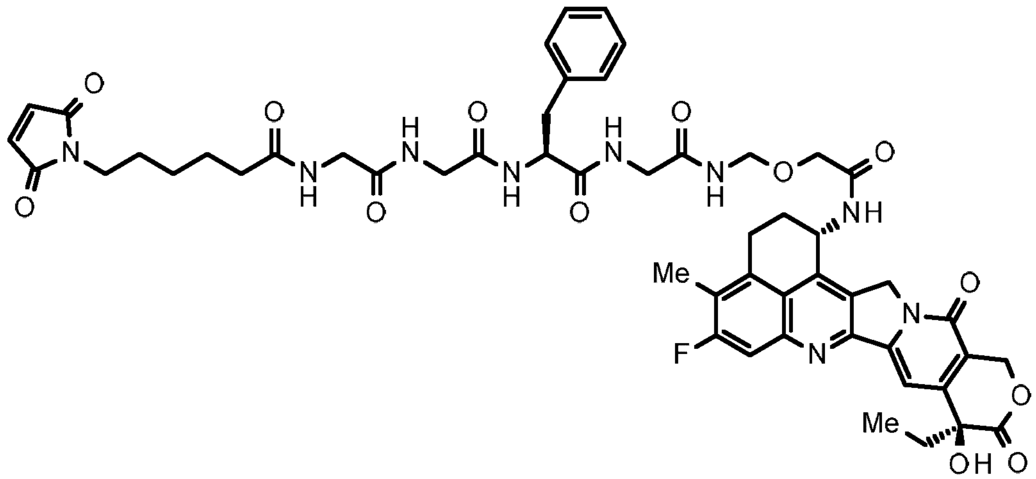

was added thereto to react with the antibody having thiol groups. Thereby, an anti-TROP2 antibody-drug conjugate composition in which a drug-linker represented by the formula:

[Formula 17]

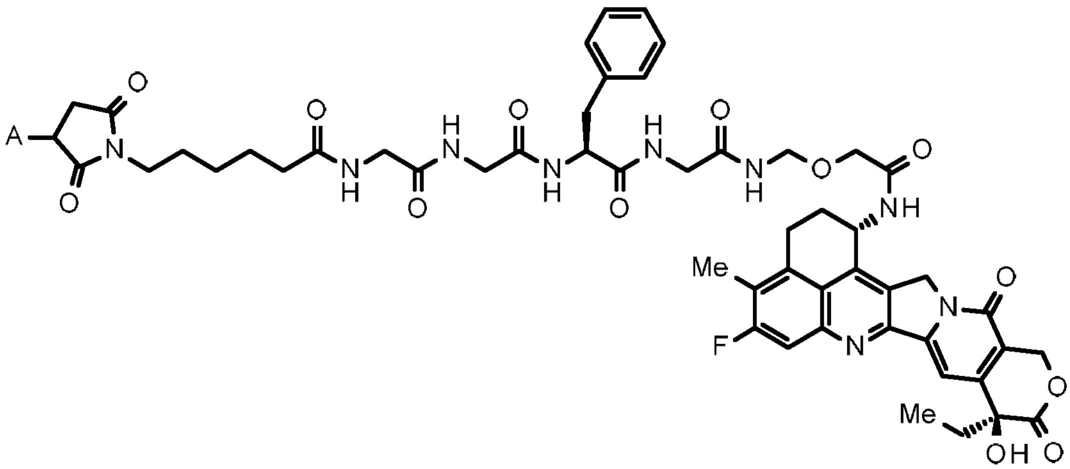

wherein A represents the connecting position to the antibody,
is conjugated to the anti-TROP2 antibody via a thioether bond was obtained.

The content of D4 in D0 to D8, and the composition ratios of D4-1 and D4-2 in D4 in the anti-TROP2 antibody-drug conjugate composition obtained were measured to track the temporal variation of the reaction.

The results at a reaction temperature of 0°C are shown in Table 1.

[Table 1]

| Time | Content of D4 (%) | Composition ratio (%) | |
|---|---|---|---|
| | | D4-1 | D4-2 |
| 1 | 50 | 95 | 5 |
| 2 | 55 | 94 | 6 |
| 3 | 56 | 93 | 7 |
| 4 | 59 | 92 | 8 |

(continued)

| Time | Content of D4 (%) | Composition ratio (%) | |
|---|---|---|---|
| | | D4-1 | D4-2 |
| 5 | 58 | 92 | 8 |
| 6 | 58 | 91 | 9 |
| 7 | 59 | 89 | 11 |
| 22 | 58 | 78 | 22 |
| 25 | 58 | 77 | 23 |
| 31 | 58 | 74 | 26 |
| 48 | 58 | 70 | 30 |
| 50 | 59 | 70 | 30 |

The composition ratio of D4-1 decreased as the reaction time became longer, and the composition ratio of D4-2 increased as the reaction time became longer. Further, it was found that the composition ratio of D4-1 and the composition ratio of D4-2 each came to a constant value after a reaction time of 31 hours or more (preferably 48 hours or more). This confirmed that in the case of a reaction temperature of 0°C, the composition ratio of the antibody having four heavy-light interchain thiols and the composition ratio of the antibody having four heavy-heavy interchain thiols each reaches a steady value in 31 hours or more (preferably 48 hours or more).

The results at a reaction temperature of 10°C are shown in Table 2.

[Table 2]

| Time | Content of D4 (%) | Composition ratio (%) | |
|---|---|---|---|
| | | D4-1 | D4-2 |
| 1 | 56 | 93 | 7 |
| 2 | 58 | 89 | 11 |
| 3 | 58 | 86 | 14 |
| 5 | 58 | 80 | 20 |
| 6 | 57 | 77 | 23 |
| 20 | 57 | 66 | 34 |
| 24 | 57 | 66 | 34 |
| 28 | 57 | 64 | 36 |
| 44 | 56 | 65 | 35 |
| 51 | 55 | 65 | 35 |

The composition ratio of D4-1 decreased as the reaction time became longer, and the composition ratio of D4-2 increased as the reaction time became longer. Further, it was found that the composition ratio of D4-1 and the composition ratio of D4-2 each came to a constant value after a reaction time of 20 hours or more. This confirmed that in the case of a reaction temperature of 10°C, the composition ratio of the antibody having four heavy-light interchain thiols and the composition ratio of the antibody having four heavy-heavy interchain thiols each reaches a steady value in 20 hours or more.

The results at a reaction temperature of 20°C are shown in Table 3.

[Table 3]

| Time | Content of D4 (%) | Composition ratio (%) | |
|---|---|---|---|
| | | D4-1 | D4-2 |
| 1 | 55 | 84 | 16 |
| 2 | 56 | 75 | 25 |
| 3 | 55 | 69 | 31 |
| 4 | 55 | 66 | 34 |
| 5 | 56 | 65 | 35 |
| 6 | 55 | 63 | 37 |
| 7 | 55 | 63 | 37 |
| 22 | 54 | 62 | 38 |
| 25 | 54 | 62 | 38 |
| 31 | 54 | 62 | 38 |
| 48 | 52 | 63 | 37 |
| 50 | 52 | 63 | 37 |

The content of D4 was somewhat lower than those at 0°C and 10°C, and found to tend to slightly decrease as the reaction time became longer. The composition ratio of D4-1 decreased as the reaction time became longer, and the composition ratio of D4-2 increased as the reaction time became longer. Further, it was found that the composition ratio of D4-1 and the composition ratio of D4-2 each came to a constant value after a reaction time of 4 hours or more (preferably 5 hours or more, more preferably 6 hours or more). This confirmed that in the case of a reaction temperature of 20°C, the composition ratio of the antibody having four heavy-light interchain thiols and the composition ratio of the antibody having four heavy-heavy interchain thiols each reaches a steady value in 4 hours or more (preferably 5 hours or more, more preferably 6 hours or more).

As the result for reaction temperatures 0°C, 10°C, and 20°C, the temporal variation of the content of D4 in D0 to D8 is shown in Figure 5, and the temporal variations of the composition ratios of D4-1 and D4-2 in D4 are shown in Figure 6.

[Example 2] Tracking of temporal variations of composition ratios of D4-1 and D4-2 in production of anti-B7-H3 antibody-drug conjugate composition

A solution containing an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4) (corresponding to 250 mg of the antibody) was placed in a polypropylene tube, to which polysorbate 20 (2.9 mg), 0.5 mol/L aqueous solution of EDTA (6 equivalents to the antibody), and 0.05 mol/L aqueous solution of L-histidine (12.5 mL) were further added. Subsequently, an aqueous solution containing tris(2-carboxyethyl)phosphine hydrochloride (1.03 mg; 2.1 equivalents per antibody molecule) was added under stirring at 5°C or 10°C, and shaking was performed at the same temperature to form an antibody having thiol groups. After the lapse of 1 hour, 2 hours, 3 hours, 4 hours, 6 hours, 8 hours, 12 hours, 16 hours, 20 hours, 24 hours, 36 hours, or 48 hours, a part of the reaction solution was collected and a compound represented by the formula:

[Formula 18]

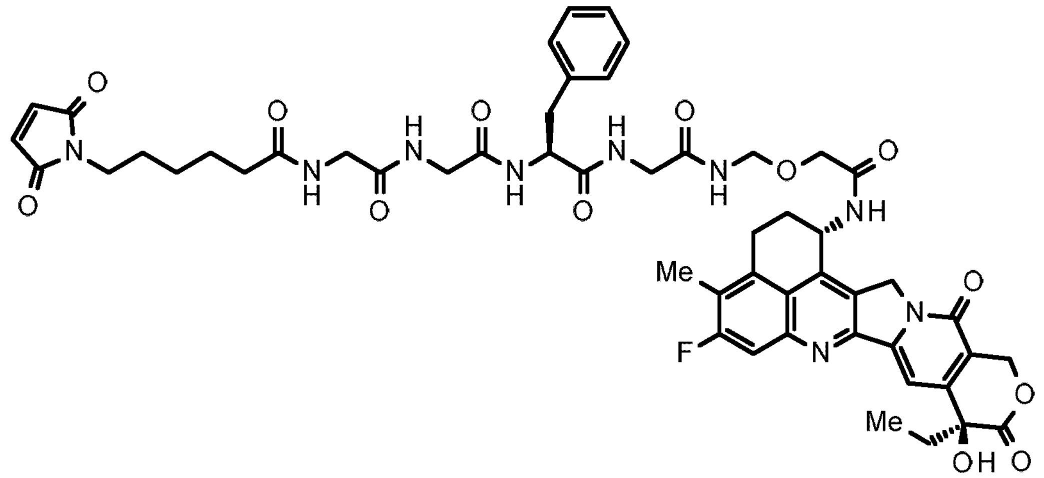

was added thereto to react with the antibody having thiol groups. Thereby, an anti-B7-H3 antibody-drug conjugate composition in which a drug-linker represented by the formula:

[Formula 19]

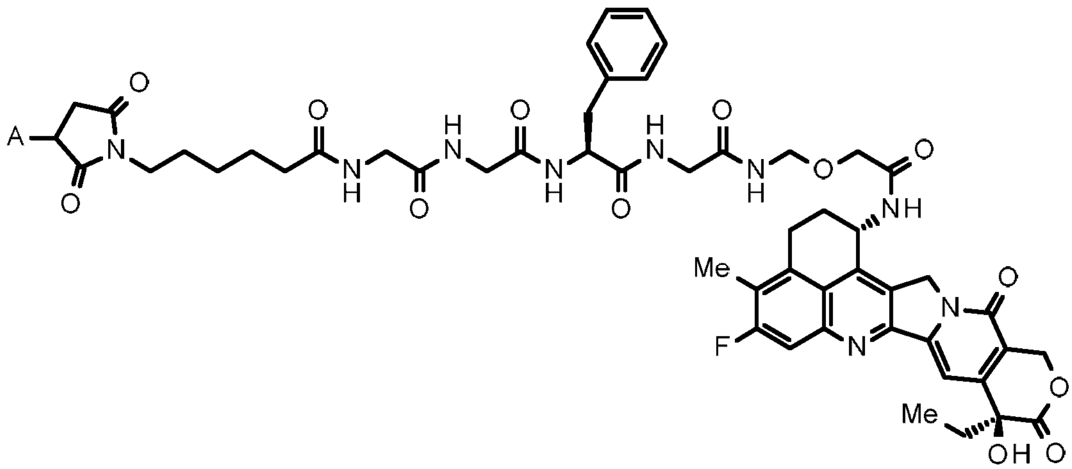

wherein A represents the connecting position to the antibody,
is conjugated to the anti-B7-H3 antibody via a thioether bond was obtained.

The content of D4 in D0 to D8, and the composition ratios of D4-1 and D4-2 in D4 in the anti-B7-H3 antibody-drug conjugate composition obtained were measured to track the temporal variation of the reaction.
The results at a reaction temperature of 5°C are shown in Table 4.

[Table 4]

| Time | Content of D4 (%) | Composition ratio (%) | |
|---|---|---|---|
| | | D4-1 | D4-2 |
| 1 | 53 | 94 | 6 |
| 2 | 57 | 92 | 8 |
| 3 | 58 | 89 | 11 |

(continued)

| Time | Content of D4 (%) | Composition ratio (%) | |
|---|---|---|---|
| | | D4-1 | D4-2 |
| 4 | 58 | 87 | 13 |
| 6 | 58 | 82 | 18 |
| 8 | 57 | 78 | 22 |
| 12 | 57 | 73 | 27 |
| 16 | 56 | 70 | 30 |
| 20 | 56 | 69 | 31 |
| 24 | 55 | 68 | 32 |
| 36 | 54 | 66 | 34 |
| 48 | 53 | 65 | 35 |

The composition ratio of D4-1 decreased as the reaction time became longer, and the composition ratio of D4-2 increased as the reaction time became longer. Further, it was found that the composition ratio of D4-1 and the composition ratio of D4-2 each came to a constant value after a reaction time of 16 hours or more (preferably 20 hours or more, more preferably 24 hours or more (or 36 hours or more)). This confirmed that in the case of a reaction temperature of 5°C, the composition ratio of the antibody having four heavy-light interchain thiols and the composition ratio of the antibody having four heavy-heavy interchain thiols each reaches a steady value in 16 hours or more (preferably 20 hours or more, more preferably 24 hours or more (or 36 hours or more)) .

The results at a reaction temperature of 10°C are shown in Table 5.

[Table 5]

| Time | Content of D4 (%) | Composition ratio (%) | |
|---|---|---|---|
| | | D4-1 | D4-2 |
| 1 | 55 | 91 | 9 |
| 2 | 57 | 87 | 13 |
| 3 | 57 | 82 | 18 |
| 4 | 56 | 78 | 22 |
| 6 | 56 | 73 | 27 |
| 8 | 55 | 70 | 30 |
| 12 | 55 | 65 | 35 |
| 16 | 55 | 64 | 36 |
| 20 | 54 | 64 | 36 |
| 24 | 54 | 63 | 37 |
| 36 | 52 | 65 | 35 |
| 48 | 51 | 63 | 37 |

The composition ratio of D4-1 decreased as the reaction time became longer, and the composition ratio of D4-2 increased as the reaction time became longer. Further, it was found that the composition ratio of D4-1 and the composition ratio of D4-2 each came to a constant value after a reaction time of 12 hours or more. This confirmed that in the case of a reaction temperature of 10°C, the composition ratio of the antibody having four heavy-light interchain thiols and the composition ratio of the antibody having four heavy-heavy interchain thiols each reaches a steady value in 12 hours or

more.

As the result for reaction temperatures 5°C and 10°C, the temporal variation of the content of D4 in D0 to D8 is shown in Figure 7, and the temporal variations of the composition ratios of D4-1 and D4-2 in D4 are shown in Figure 8.

[Example 3] Production of anti-TROP2 antibody-drug conjugate composition with method of present invention

A solution containing an anti-TROP2 antibody (an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2) (liquid weight: 10.4 kg, corresponding to 400 g of the antibody) was placed in a single-use reactor, to which 0.1 g/g aqueous solution of polysorbate 80 (40 mL), 0.5 mol/L aqueous solution of EDTA (28 mL; 5 equivalents to the antibody), and 0.01 mol/L aqueous solution of L-histidine (30 kg) were further added, and 0.3 mol/L aqueous solution of disodium hydrogen phosphate was then added thereto to adjust the pH to 6.9. An aqueous solution containing 1 mg/g tris(2-carboxyethyl)phosphine hydrochloride (1.70 kg; 2.15 equivalents per antibody molecule) was added under stirring at 10 to 11°C for reduction, and stirring was performed at an inner temperature of 10 to 11°C for 30 hours to control the composition ratio of the antibody having four heavy-light interchain thiols and the composition ratio of the antibody having four heavy-heavy interchain thiols to reach steady values.

To the resulting reaction solution, a compound (13.4 g; 4.4 equivalents per antibody molecule) represented by the formula:

[Formula 20]

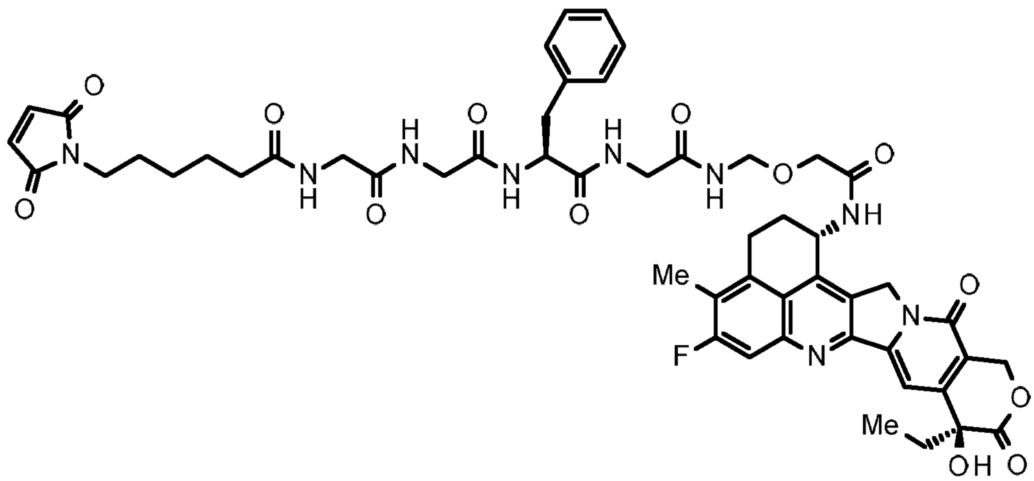

, which had been dissolved in a mixture of 10% aqueous solution of acetic acid (7.4 mL) and 80% aqueous solution of dimethyl sulfoxide (1.5 L), was added under stirring at an inner temperature of 10°C over 26 minutes, and stirring was performed at the same temperature for 50 minutes for conjugation to the antibody having thiol groups. Subsequently, 0.05 M aqueous solution of N-acetylcysteine (0.17 L; 3 equivalents per antibody molecule) was added, and stirring was further performed at the same temperature for 1 hour, and the pH was then adjusted to 5 with 10% aqueous solution of acetic acid.

The resulting solution was subjected to ultrafiltration using a Pellicon (R) 2 Ultracel (R) (manufactured by Merck KGaA) by adding and circulating 0.01 mol/L histidine buffer solution (pH 5) with a roller pump to remove impurities derived from the compound. Concentration of the solution and adjustment of the pH to 6 were carried out to afford 7.00 kg of a solution containing an anti-TROP2 antibody-drug conjugate composition in which a drug-linker represented by the formula:

[Formula 21]

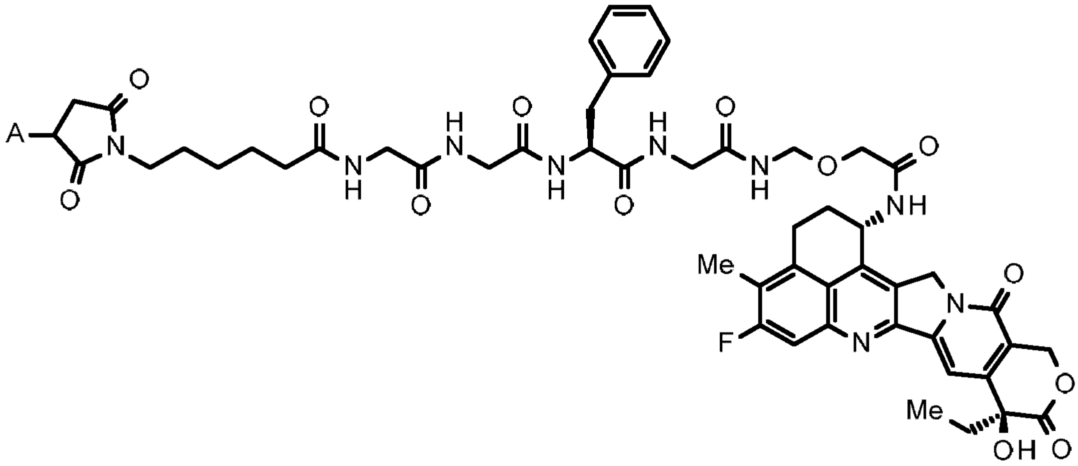

wherein A represents the connecting position to the antibody,
is conjugated to the anti-TROP2 antibody via a thioether bond.

To the solution, a histidine buffer solution dissolving sucrose (1.6 kg) therein at pH 6, a histidine buffer solution at pH 6, and 0.1 g/L aqueous solution of polysorbate 80 (28 g) were further added, and additionally 9% sucrose-containing histidine buffer solution (pH 6) was added to adjust the protein concentration to 20 g/L, and thus a pharmaceutical composition containing the anti-TROP2 antibody-drug conjugate composition (19.0 kg) was obtained. The pharmaceutical composition obtained was such that protein concentration: 20.3 g/L, protein yield: 374 g, average number of conjugated drug molecules per antibody molecule: 4.0. The contents of the isomers were D0: 3%, D2: 18%, D4: 53% (in which D4-1: 60%, D4-2: 40%), D6: 20%, D8: 4%.

[Example 4] Production of anti-B7-H3 antibody-drug conjugate composition with method of present invention

A solution containing an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4) (liquid weight: 10.2 kg, corresponding to 200 g of the antibody) was placed in a single-use reactor, to which polysorbate 20 (4.6 g), 0.5 mol/L aqueous solution of EDTA (18 g; 6 equivalents to the antibody), and 0.05 mol/L aqueous solution of L-histidine (10 kg) were further added. An aqueous solution containing tris(2-carboxyethyl)phosphine hydrochloride (0.831 g; 2.1 equivalents per antibody molecule) was added under stirring at 5°C for reduction, and stirring was performed at an inner temperature of 5°C for 4 hours and at an inner temperature of 10°C for 15 hours to control the composition ratio of the antibody having four heavy-light interchain thiols and the composition ratio of the antibody having four heavy-heavy interchain thiols to reach steady values.

To the resulting reaction solution, a compound (6.64 g; 4.4 equivalents per antibody molecule) represented by the formula:

[Formula 22]

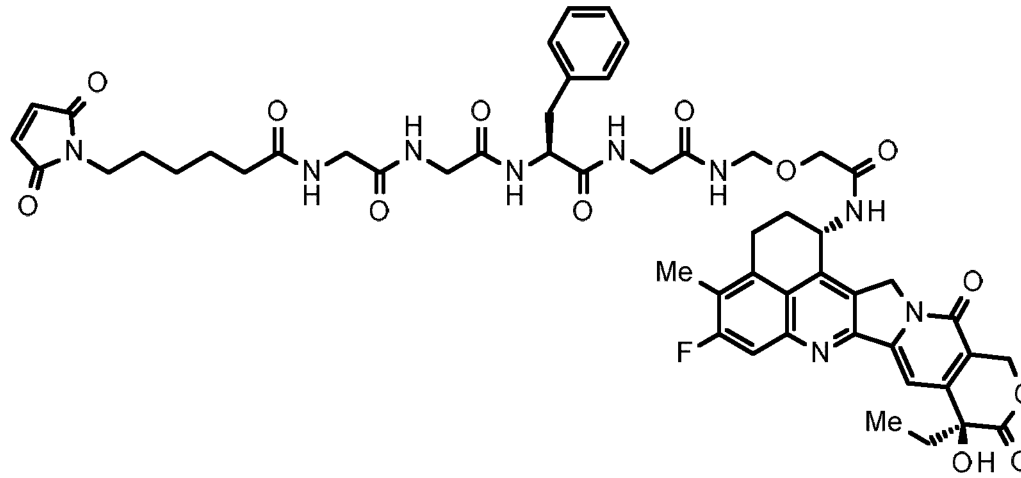

, which had been dissolved in 80% aqueous solution of dimethyl sulfoxide (1.1 kg) containing 10% aqueous solution of acetic acid (3.7 mL), was added under stirring at an inner temperature of 9 to 11°C over 50 minutes, and stirring was performed at the same temperature for 24 minutes for conjugation to the antibody having thiol groups. Subsequently, 0.05 M aqueous solution of N-acetylcysteine (0.14 kg; 5 equivalents per antibody molecule) was added, and stirring was further performed at the same temperature for 1 hour, and the pH was then adjusted to 5 with 10% aqueous solution of acetic acid.

The resulting solution was subjected to ultrafiltration using a Pellicon (R) 2 Ultracel (R) (manufactured by Merck KGaA) by adding and circulating 0.01 mol/L histidine buffer solution (pH 5) with a roller pump to remove impurities derived from the compound. Concentration of the solution and adjustment of the pH to 5.9 were carried out to afford 6.82 kg of a solution containing an anti-B7-H3 antibody-drug conjugate composition in which a drug-linker represented by the formula:

[Formula 23]

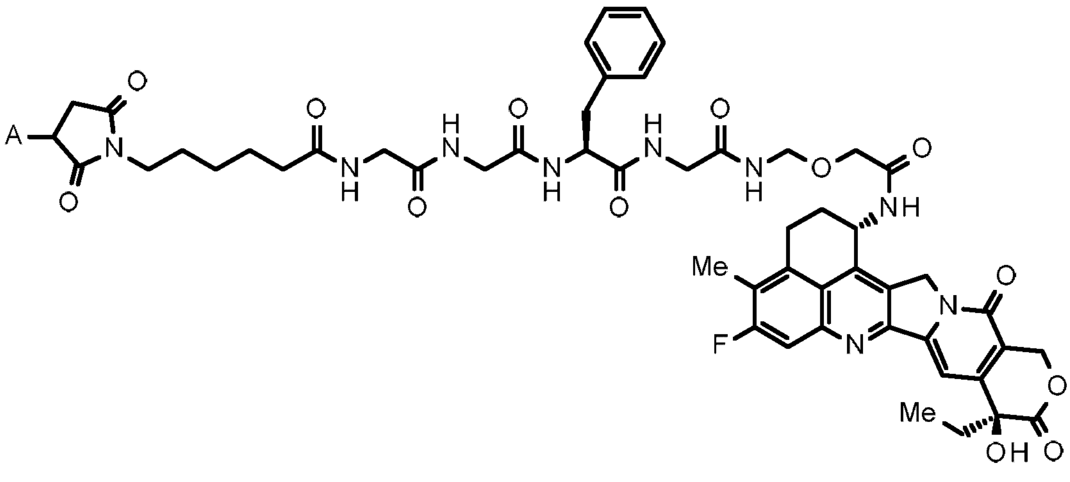

wherein A represents the connecting position to the antibody,
is conjugated to the anti-B7-H3 antibody via a thioether bond.

To the solution, histidine buffer solution dissolving sucrose (0.83 kg) therein at pH 5.9 and histidine buffer solution at pH 5.9 were further added, and additionally 9% sucrose-containing histidine buffer solution (pH 5.9) was added to adjust the protein concentration to 20 g/L, and thus a pharmaceutical composition containing the anti-B7-H3 antibody-drug conjugate composition (10.1 kg) was obtained. The pharmaceutical composition obtained was such that protein concentration: 20.4 g/L, protein yield: 198 g, average number of conjugated drug molecules per antibody molecule:

4.0. The contents of the isomers were D0: 3%, D2: 20%, D4: 54% (in which D4-1: 64%, D4-2: 36%), D6: 18%, D8: 5%.

[Example 5] Production of anti-B7-H3 antibody-drug conjugate composition with method of present invention

To a solution containing an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4) (liquid weight: 51.0 g, corresponding to 1.0 g of the antibody), 100 mg/g aqueous solution of polysorbate 20 (0.23 mL), 0.5 mol/L aqueous solution of EDTA (6 equivalents to the antibody), and 0.05 mol/L aqueous solution of L-histidine (50 mL) were added. An aqueous solution containing tris(2-carboxyethyl)phosphine hydrochloride (4.50 mg; 2.3 equivalents per antibody molecule) was added under stirring at 5°C for reduction, and stirring was performed at an inner temperature of 5°C for 24 hours to control the composition ratio of the antibody having four heavy-light interchain thiols and the composition ratio of the antibody having four heavy-heavy interchain thiols to reach steady values.

To the resulting reaction solution, a compound (33 mg; 4.4 equivalents per antibody molecule) represented by the formula:

[Formula 24]

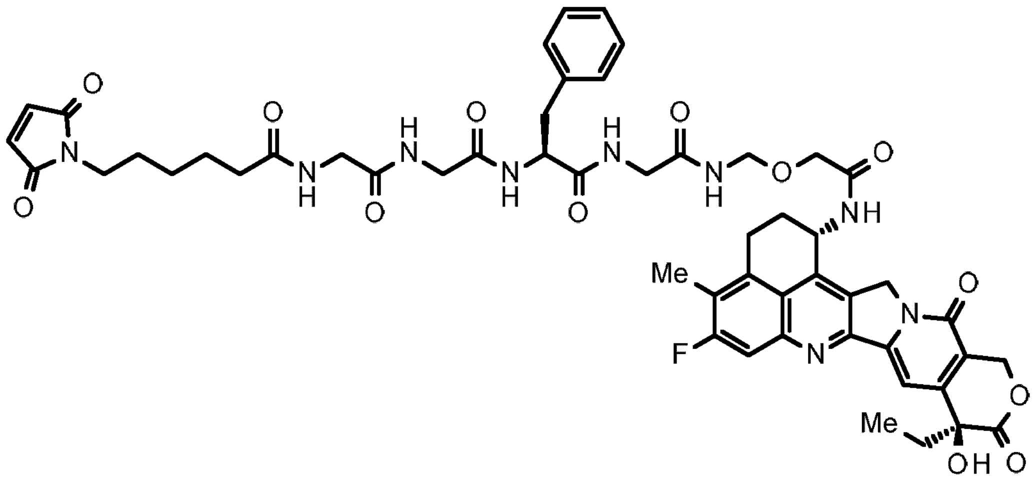

, which had been dissolved in 80% aqueous solution of dimethyl sulfoxide (5.5 g) containing 10% aqueous solution of acetic acid (0.018 mL), was added under stirring at an inner temperature of 4 to 6°C over 20 minutes, and stirring was performed at the same temperature for 60 minutes for conjugation to the antibody having thiol groups. Subsequently, 0.05 M aqueous solution of N-acetylcysteine (0.68 g; 5 equivalents per antibody molecule) was added, and stirring was further performed at the same temperature for 1 hour, and the pH was then adjusted to 5 with 10% aqueous solution of acetic acid to afford a solution containing an anti-B7-H3 antibody-drug conjugate composition in which a drug-linker represented by the formula:

[Formula 25]

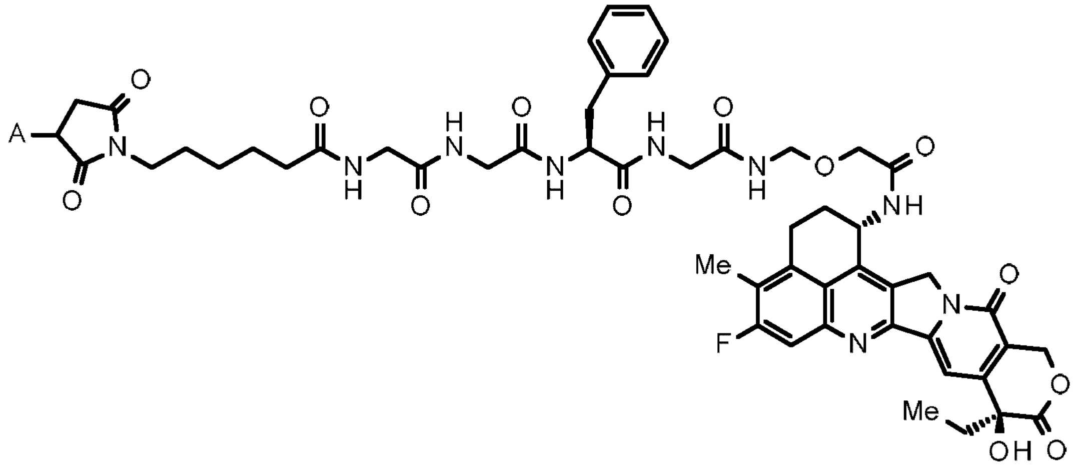

wherein A represents the connecting position to the antibody, is conjugated to the anti-B7-H3 antibody via a thioether bond. The contents of the isomers in the solution were D0: 4%, D2: 15%, D4: 54% (in which D4-1: 65%, D4-2: 35%), D6: 21%, D8: 5%.

[Example 6] Production of anti-B7-H3 antibody-drug conjugate composition with method of present invention

To a solution containing an anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4) (liquid weight: 30.6 g, corresponding to 600 mg of the antibody), 100 mg/g aqueous solution of polysorbate 20 (0.14 mL), 0.5 mol/L aqueous solution of EDTA (6 equivalents to the antibody), and 0.05 mol/L aqueous solution of L-histidine (30 mL) were added. An aqueous solution containing tris(2-carboxyethyl)phosphine hydrochloride (2.47 mg; 2.1 equivalents per antibody molecule) was added under stirring at 10°C for reduction, and stirring was performed at an inner temperature of 10°C for 24 hours to control the composition ratio of the antibody having four heavy-light interchain thiols and the composition ratio of the antibody having four heavy-heavy interchain thiols to reach steady values.

To the resulting reaction solution, a compound (20 mg; 4.4 equivalents per antibody molecule) represented by the formula:

[Formula 26]

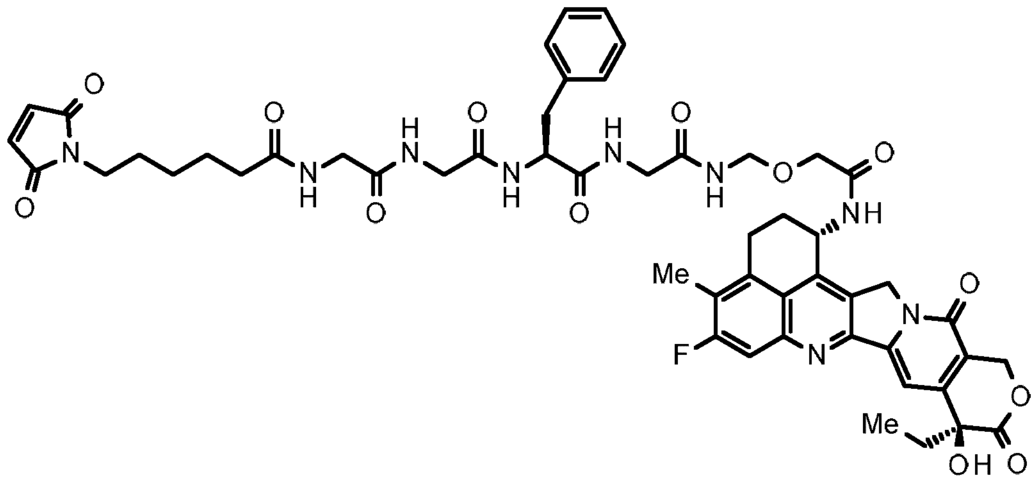

, which had been dissolved in 80% aqueous solution of dimethyl sulfoxide (3.3 g) containing 10% aqueous solution

of acetic acid (0.011 mL), was added under stirring at an inner temperature of 9 to 11°C over 20 minutes, and stirring was performed at the same temperature for 45 minutes for conjugation to the antibody having thiol groups. Subsequently, 0.05 M aqueous solution of N-acetylcysteine (0.41 g; 5 equivalents per antibody molecule) was added, and stirring was further performed at the same temperature for 45 minutes, and the pH was then adjusted to 5 with 10% aqueous solution of acetic acid to afford a solution containing an anti-B7-H3 antibody-drug conjugate composition in which a drug-linker represented by the formula:

[Formula 27]

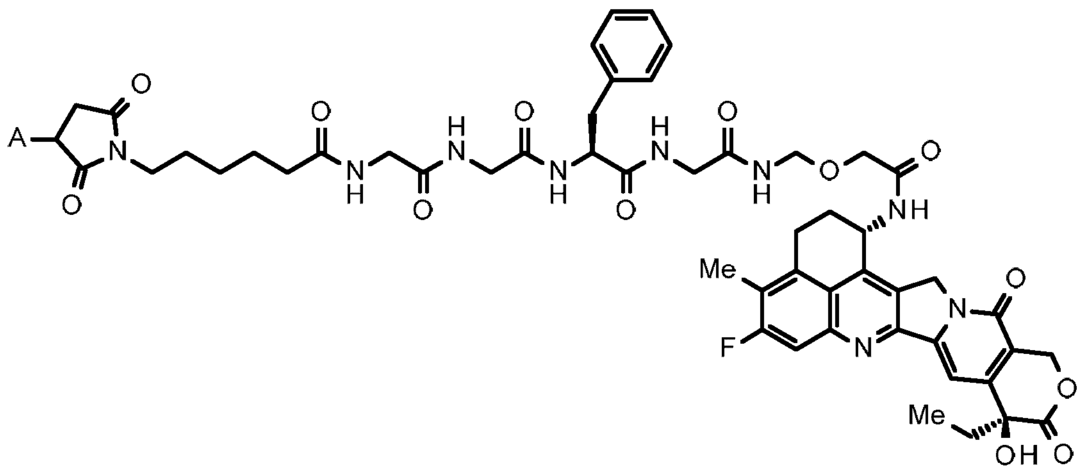

wherein A represents the connecting position to the antibody,
is conjugated to the anti-B7-H3 antibody via a thioether bond. The contents of the isomers in the solution were D0: 4%, D2: 22%, D4: 54% (in which D4-1: 64%, D4-2: 36%), D6: 17%, D8: 3%.

Free Text of Sequence Listing

SEQ ID NO: 1 - Amino acid sequence of a heavy chain of the anti-TROP2 antibody
SEQ ID NO: 2 - Amino acid sequence of a light chain of the anti-TROP2 antibody
SEQ ID NO: 3 - Amino acid sequence of a heavy chain of the anti-B7-H3 antibody
SEQ ID NO: 4 - Amino acid sequence of a light chain of the anti-B7-H3 antibody
SEQ ID NO: 5 - Amino acid sequence of CDRH1 of the anti-TROP2 antibody
SEQ ID NO: 6 - Amino acid sequence of CDRH2 of the anti-TROP2 antibody
SEQ ID NO: 7 - Amino acid sequence of CDRH3 of the anti-TROP2 antibody
SEQ ID NO: 8 - Amino acid sequence of CDRL1 of the anti-TROP2 antibody
SEQ ID NO: 9 - Amino acid sequence of CDRL2 of the anti-TROP2 antibody
SEQ ID NO: 10 - Amino acid sequence of CDRL3 of the anti-TROP2 antibody
SEQ ID NO: 11 - Amino acid sequence of CDRH1 of the anti-B7-H3 antibody
SEQ ID NO: 12 - Amino acid sequence of CDRH2 of the anti-B7-H3 antibody
SEQ ID NO: 13 - Amino acid sequence of CDRH3 of the anti-B7-H3 antibody
SEQ ID NO: 14 - Amino acid sequence of CDRL1 of the anti-B7-H3 antibody
SEQ ID NO: 15 - Amino acid sequence of CDRL2 of the anti-B7-H3 antibody
SEQ ID NO: 16 - Amino acid sequence of CDRL3 of the anti-B7-H3 antibody

## Claims

**1.** A method for producing an antibody-drug conjugate composition, comprising:

(i) a step of reacting an antibody with a reducing agent to obtain an antibody having thiol groups; then
(ii) a step of reacting drug-linker intermediates with the antibody having thiol groups obtained in the step (i),

wherein

the step (i) is carried out until the composition ratio of the antibody having four heavy-light interchain thiols and the composition ratio of the antibody having four heavy-heavy interchain thiols reach a steady value.

2. The production method according to claim 1, wherein the step (i) is carried out for 4 hours or more.

3. The production method according to claim 1, wherein the step (i) is carried out for 12 hours or more.

4. The production method according to claim 1, wherein the step (i) is carried out for 16 hours or more.

5. The production method according to claim 1, wherein the step (i) is carried out for 20 hours or more.

6. The production method according to claim 1, wherein the step (i) is carried out for 31 hours or more.

7. The production method according to any one of claims 1 to 6, wherein the step (i) is carried out at 0 to 20°C.

8. The production method according to any one of claims 1 to 6, wherein the step (i) is carried out at 5 to 20°C.

9. The production method according to any one of claims 1 to 6, wherein the step (i) is carried out at 5 to 10°C.

10. The production method according to any one of claims 1 to 6, wherein the step (i) is carried out at about 10°C.

11. The production method according to any one of claims 1 to 10, wherein the average number of units of the drug-linker conjugated per antibody molecule in the produced antibody-drug conjugate composition is in the range of from 3.5 to 4.5.

12. The production method according to any one of claims 1 to 11, wherein the content of the antibody-drug conjugates in which four drug-linkers are conjugated, in the produced antibody-drug conjugate composition is 50% or more.

13. The production method according to any one of claims 1 to 12, wherein the composition ratio of the antibody-drug conjugates in which four drug-linkers are conjugated to heavy-light interchain thiols is 1.5 to 2.5 times the composition ratio of the antibody-drug conjugates in which four drug-linkers are conjugated to heavy-heavy interchain thiols.

14. The production method according to any one of claims 1 to 13, wherein the reducing agent is used at 1.9 to 2.5 equivalents per molecule of antibody.

15. The production method according to any one of claims 1 to 14, wherein the reducing agent is tris(2-carboxyethyl)phosphine or a salt thereof.

16. The production method according to any one of claims 1 to 15, wherein the drug-linker intermediate has an N-substituted maleimidyl group.

17. The production method according to any one of claims 1 to 15, wherein the drug-linker intermediate is a compound represented by the following formula:

[Formula 1]

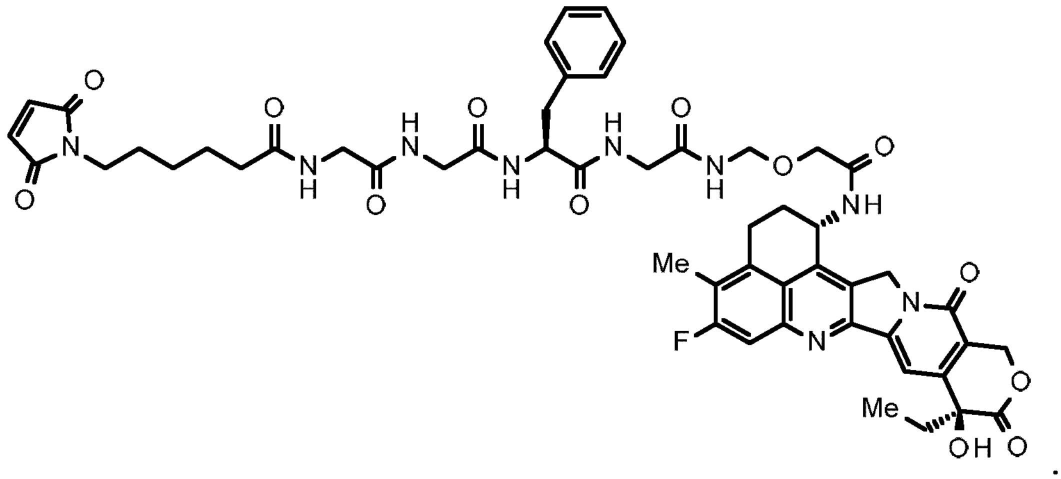

18. The production method according to claim 17, wherein the drug-linker in the produced antibody-drug conjugate composition is represented by the following formula:

[Formula 2]

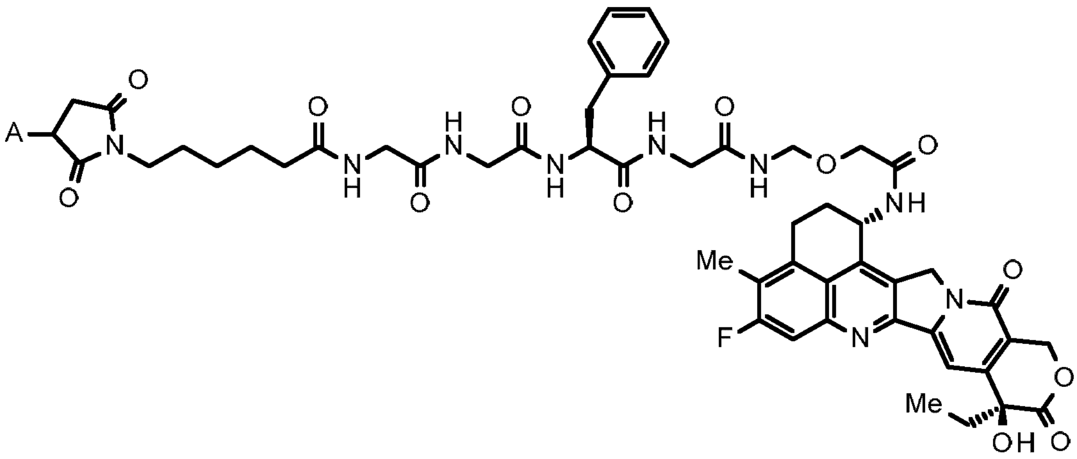

wherein A represents the connecting position to the antibody, and the drug-linker is conjugated to the antibody via a thioether bond.

19. The production method according to any one of claims 1 to 18, wherein the antibody is an anti-TROP2 antibody or an anti-B7-H3 antibody.

20. The production method according to claim 19, wherein the antibody is an anti-TROP2 antibody.

21. The production method according to claim 20, wherein the anti-TROP2 antibody is an antibody comprising a heavy chain comprising CDRH1 consisting of an amino acid sequence represented by SEQ ID NO: 5, CDRH2 consisting of an amino acid sequence represented by SEQ ID NO: 6 and CDRH3 consisting of an amino acid sequence represented by SEQ ID NO: 7, and a light chain comprising CDRL1 consisting of an amino acid sequence represented by SEQ ID NO: 8, CDRL2 consisting of an amino acid sequence represented by SEQ ID NO: 9 and CDRL3 consisting of an amino acid sequence represented by SEQ ID NO: 10.

22. The production method according to claim 20, wherein the anti-TROP2 antibody is an antibody comprising a heavy chain comprising a heavy chain variable region consisting of an amino acid sequence consisting of amino acid residues 20 to 140 of SEQ ID NO: 1 and a light chain comprising a light chain variable region consisting of an amino acid sequence consisting of amino acid residues 21 to 129 of SEQ ID NO: 2.

**23.** The production method according to claim 20, wherein the anti-TROP2 antibody is an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2.

**24.** The production method according to claim 23, wherein the anti-TROP2 antibody lacks a lysine residue at the carboxyl terminus of the heavy chain.

**25.** The production method according to claim 19, wherein the antibody is an anti-B7-H3 antibody.

**26.** The production method according to claim 25, wherein the anti-B7-H3 antibody is an antibody comprising a heavy chain comprising CDRH1 consisting of an amino acid sequence represented by SEQ ID NO: 11, CDRH2 consisting of an amino acid sequence represented by SEQ ID NO: 12 and CDRH3 consisting of an amino acid sequence represented by SEQ ID NO: 13, and a light chain comprising CDRL1 consisting of an amino acid sequence represented by SEQ ID NO: 14, CDRL2 consisting of an amino acid sequence represented by SEQ ID NO: 15 and CDRL3 consisting of an amino acid sequence represented by SEQ ID NO: 16.

**27.** The production method according to claim 25, wherein the anti-B7-H3 antibody is an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

**28.** The production method according to claim 25, wherein the anti-B7-H3 antibody is an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**29.** The production method according to claim 28, wherein the anti-B7-H3 antibody lacks a lysine residue at the carboxyl terminus of the heavy chain.

**30.** An antibody-drug conjugate composition produced by the production method according to any one of claims 1 to 29.

**31.** A method for producing an antibody-drug conjugate composition, comprising:

(i) a step of reacting an antibody with a reducing agent to obtain an antibody having thiol groups; then
(ii) a step of reacting drug-linker intermediates with the antibody having thiol groups obtained in the step (i), wherein

the step (i) is carried out at 0 to 20°C for 4 hours or more,
the content of the antibody-drug conjugates in which four drug-linkers are conjugated, in the produced antibody-drug conjugate composition is 50% or more, and
the composition ratio of the antibody-drug conjugates in which four drug-linkers are conjugated to heavy-light interchain thiols is 1.5 to 2.5 times the composition ratio of the antibody-drug conjugates in which four drug-linkers are conjugated to heavy-heavy interchain thiols.

**32.** The production method according to claim 31, wherein the step (i) is carried out for 12 hours or more.

**33.** The production method according to claim 31, wherein the step (i) is carried out for 16 hours or more.

**34.** The production method according to claim 31, wherein the step (i) is carried out for 20 hours or more.

**35.** The production method according to claim 31, wherein the step (i) is carried out for 31 hours or more.

**36.** The production method according to any one of claims 31 to 35, wherein the step (i) is carried out at 5 to 20°C.

**37.** The production method according to any one of claims 31 to 35, wherein the step (i) is carried out at 5 to 10°C.

**38.** The production method according to any one of claims 31 to 35, wherein the step (i) is carried out at about 10°C.

**39.** The production method according to any one of claims 31 to 38, wherein the average number of units of the drug-linker conjugated per antibody molecule in the produced antibody-drug conjugate composition is in the range of from 3.5 to 4.5.

**40.** The production method according to any one of claims 31 to 39, wherein the reducing agent is used at 1.9 to 2.5 equivalents per molecule of antibody.

**41.** The production method according to any one of claims 31 to 40, wherein the reducing agent is tris(2-carboxyethyl)phosphine or a salt thereof.

**42.** The production method according to any one of claims 31 to 41, wherein the drug-linker intermediate has an N-substituted maleimidyl group.

**43.** The production method according to any one of claims 31 to 41, wherein the drug-linker intermediate is a compound represented by the following formula:

[Formula 3]

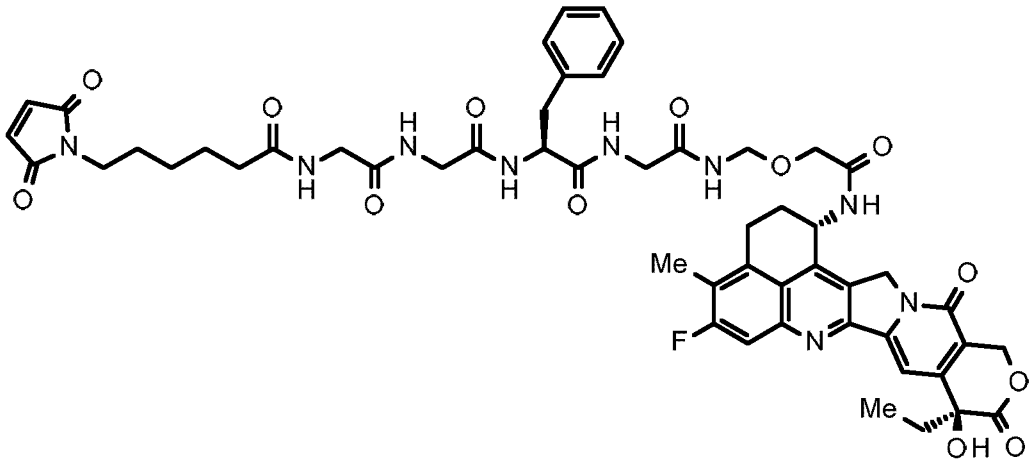

.

**44.** The production method according to claim 43, wherein the drug-linker in the produced antibody-drug conjugate composition is represented by the following formula:

[Formula 4]

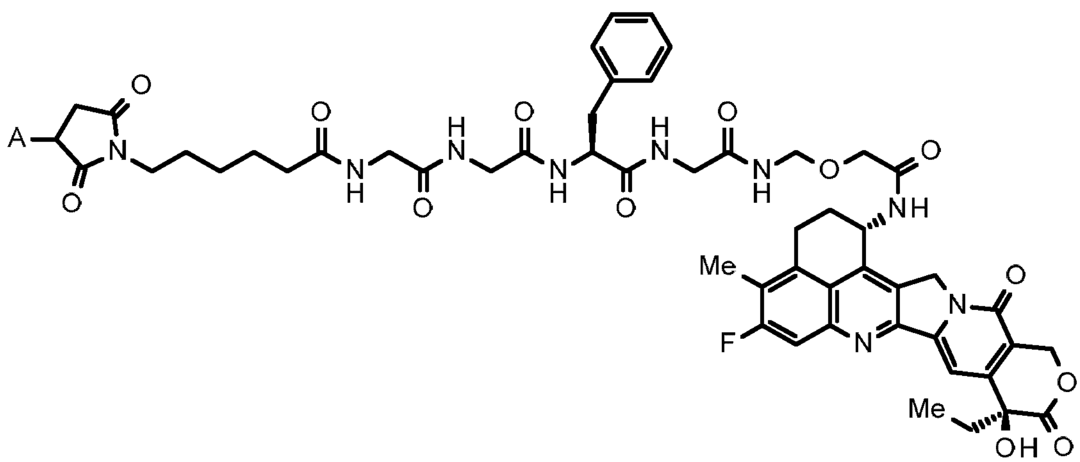

wherein A represents the connecting position to the antibody, and the drug-linker is conjugated to the antibody via a thioether bond.

**45.** The production method according to any one of claims 31 to 44, wherein the antibody is an anti-TROP2 antibody or an anti-B7-H3 antibody.

**46.** The production method according to claim 45, wherein the antibody is an anti-TROP2 antibody.

**47.** The production method according to claim 46, wherein the anti-TROP2 antibody is an antibody comprising a heavy chain comprising CDRH1 consisting of an amino acid sequence represented by SEQ ID NO: 5, CDRH2 consisting of an amino acid sequence represented by SEQ ID NO: 6 and CDRH3 consisting of an amino acid sequence represented by SEQ ID NO: 7, and a light chain comprising CDRL1 consisting of an amino acid sequence represented by SEQ ID NO: 8, CDRL2 consisting of an amino acid sequence represented by SEQ ID NO: 9 and CDRL3 consisting of an amino acid sequence represented by SEQ ID NO: 10.

**48.** The production method according to claim 46, wherein the anti-TROP2 antibody is an antibody comprising a heavy chain comprising a heavy chain variable region consisting of an amino acid sequence consisting of amino acid residues 20 to 140 of SEQ ID NO: 1 and a light chain comprising a light chain variable region consisting of an amino acid sequence consisting of amino acid residues 21 to 129 of SEQ ID NO: 2.

**49.** The production method according to claim 46, wherein the anti-TROP2 antibody is an antibody comprising a heavy chain consisting of an amino acid sequence consisting of amino acid residues 20 to 470 of SEQ ID NO: 1 and a light chain consisting of an amino acid sequence consisting of amino acid residues 21 to 234 of SEQ ID NO: 2.

**50.** The production method according to claim 49, wherein the anti-TROP2 antibody lacks a lysine residue at the carboxyl terminus of the heavy chain.

**51.** The production method according to claim 45, wherein the antibody is an anti-B7-H3 antibody.

**52.** The production method according to claim 51, wherein the anti-B7-H3 antibody is an antibody comprising a heavy chain comprising CDRH1 consisting of an amino acid sequence represented by SEQ ID NO: 11, CDRH2 consisting of an amino acid sequence represented by SEQ ID NO: 12 and CDRH3 consisting of an amino acid sequence represented by SEQ ID NO: 13, and a light chain comprising CDRL1 consisting of an amino acid sequence represented by SEQ ID NO: 14, CDRL2 consisting of an amino acid sequence represented by SEQ ID NO: 15 and CDRL3 consisting of an amino acid sequence represented by SEQ ID NO: 16.

**53.** The production method according to claim 51, wherein the anti-B7-H3 antibody is an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

**54.** The production method according to claim 51, wherein the anti-B7-H3 antibody is an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**55.** The production method according to claim 54, wherein the anti-B7-H3 antibody lacks a lysine residue at the carboxyl terminus of the heavy chain.

**56.** An antibody-drug conjugate composition produced by the production method according to any one of claims 31 to 55.

[Figure 1]

SEQ ID NO: 1 - Amino acid sequence of a heavy chain of the anti-TROP2 antibody

MKHLWFFLLLVAAPRWVLSQVQLVQSGAEVKKPGASVKVSCKASGYTFTTAGMQWVRQAPGQ
GLEWMGWINTHSGVPKYAEDFKGRVTISADTSTSTAYLQLSSLKSEDTAVYYCARSGFGSSY
WYFDVWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPGK

Signal sequence (1-19), Variable region (20-140), Constant region (141-470)

[Figure 2]

SEQ ID NO: 2 - Amino acid sequence of a light chain of the anti-TROP2 antibody

MVLQTQVFISLLLWISGAYGDIQMTQSPSSLSASVGDRVTITCKASQDVSTAVAWYQQKPGK
APKLLIYSASYRYTGVPSRFSGSGSGTDFTLTISSLQPEDFAVYYCQQHYITPLTFGQGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

Signal sequence (1-20), Variable region (21-129), Constant region (130-234)

[Figure 3]

SEQ ID NO: 3 - Amino acid sequence of a heavy chain of the anti-B7-H3 antibody

MKHLWFFLLLVAAPRWVLSQVQLVQSGAEVKKPGSSVKVSCKASGYTFTNYVMHWVRQAPGQ
GLEWMGYINPYNDDVKYNEKFKGRVTITADESTSTAYMELSSLRSEDTAVYYCARWGYYGSP
LYYFDYWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCDKTH
TCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPGK

Signal sequence (1-19), Variable region (20-141), Constant region (142-471)

[Figure 4]

SEQ ID NO: 4 - Amino acid sequence of a light chain of the anti-B7-H3 antibody

MVLQTQVFISLLLWISGAYGEIVLTQSPATLSLSPGERATLSCRASSRLIYMHWYQQKPGQA
PRPLIYATSNLASGIPARFSGSGSGTDFTLTISSLEPEDFAVYYCQQWNSNPPTFGQGTKVE
IKRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

Signal sequence (1-20), Variable region (21-128), Constant region (129-233)

[Figure 5]

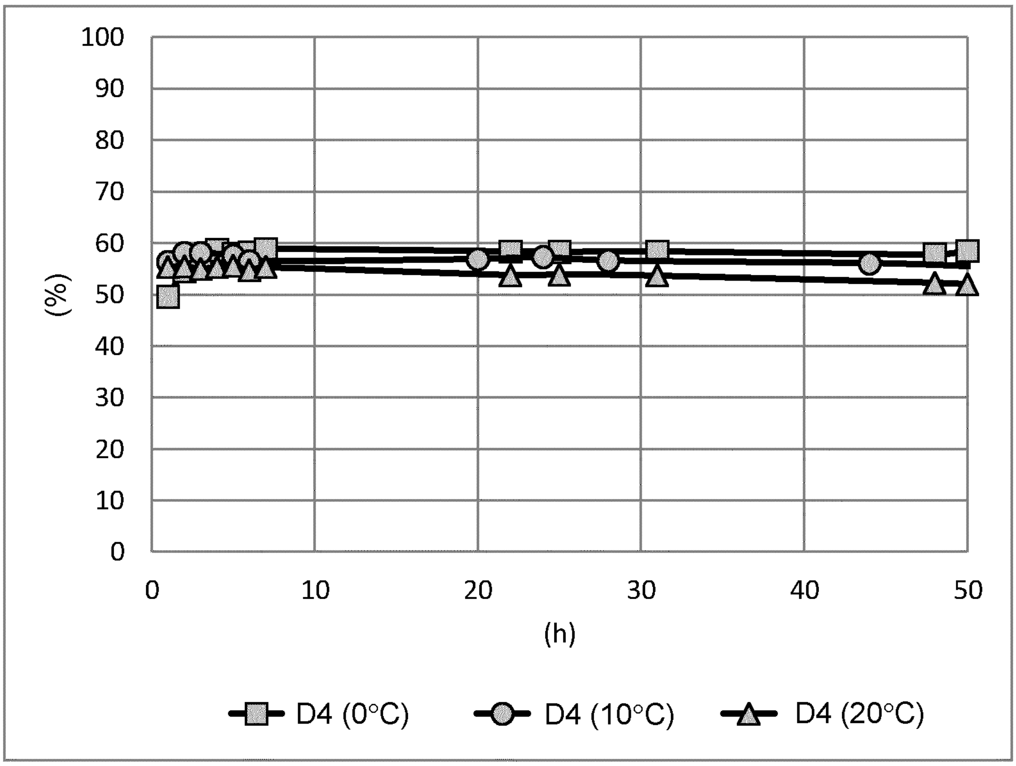

[Figure 6]

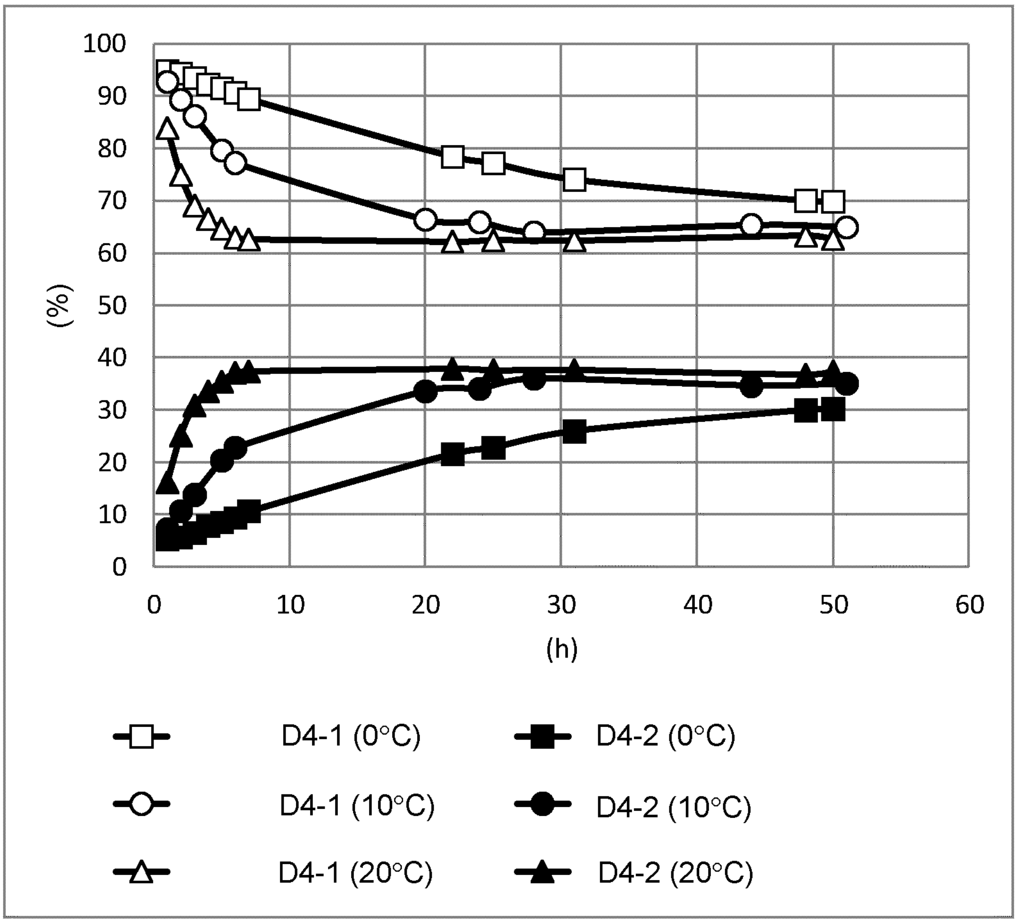
100
90
80
70
60
50
40
30
20
10
0
(%)
0
10
20
30
40
50
60
(h)
D4-1 (0°C)
D4-2 (0°C)
D4-1 (10°C)
D4-2 (10°C)
D4-1 (20°C)
D4-2 (20°C)

[Figure 7]

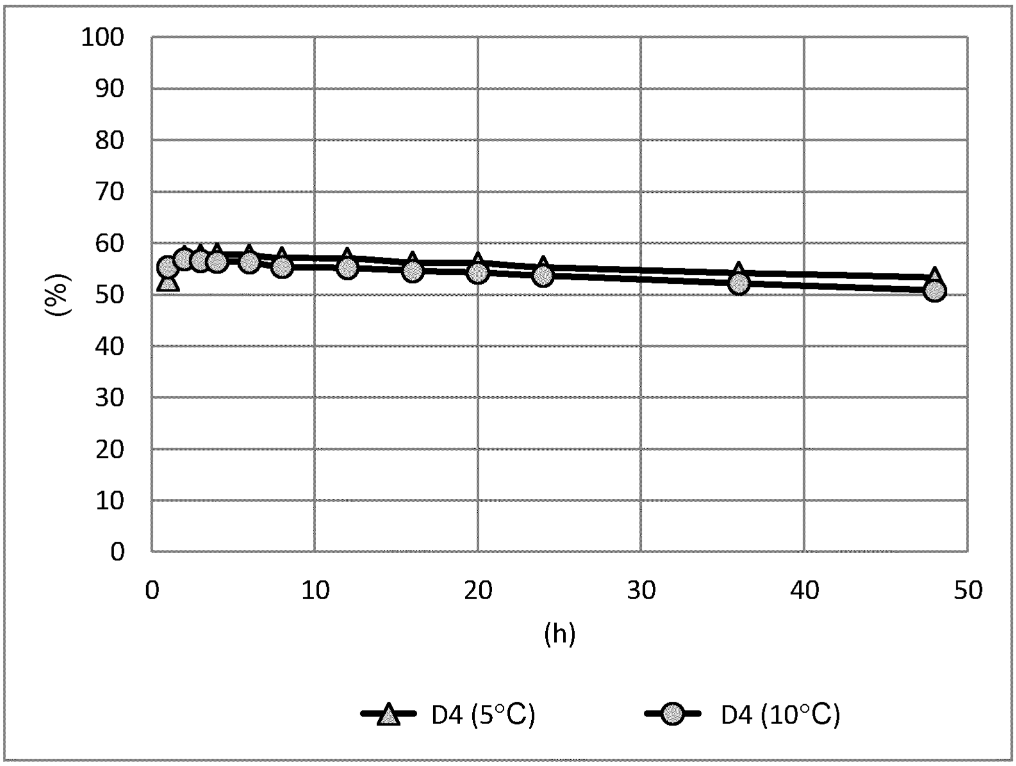

[Figure 8]

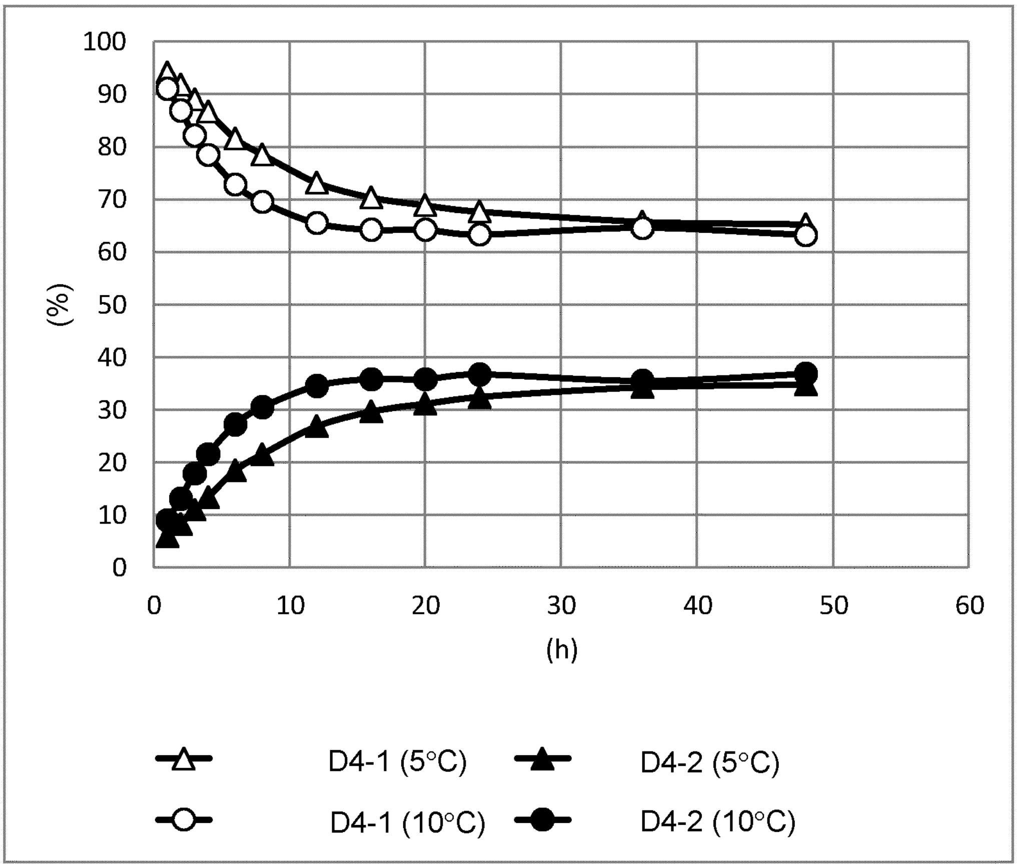
100
90
80
70
60
50
40
30
20
10
0
(%)
0
10
20
30
40
50
60
(h)
D4-1 (5°C)
D4-2 (5°C)
D4-1 (10°C)
D4-2 (10°C)

**INTERNATIONAL SEARCH REPORT**

International application No. PCT/JP2021/026784

A. CLASSIFICATION OF SUBJECT MATTER
Int. Cl. C07K16/00(2006.01)i
FI: C07K16/00 ZNA

According to International Patent Classification (IPC) or to both national classification and IPC

B. FIELDS SEARCHED

Minimum documentation searched (classification system followed by classification symbols)
Int. Cl. C07K16/00

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/MEDLINE/EMBASE/BIOSIS (STN); UniProt/GeneSeq

C. DOCUMENTS CONSIDERED TO BE RELEVANT

| Category* | Citation of document, with indication, where appropriate, of the relevant passages | Relevant to claim No. |
|---|---|---|
| A | WO 2017/002776 A1 (DAIICHI SANKYO CO., LTD.) 05 January 2017 (2017-01-05), entire text, all drawings | 1-56 |
| A | WO 2020/022363 A1 (DAIICHI SANKYO CO., LTD.) 30 January 2020 (2020-01-30), entire text, all drawings | 1-56 |
| A | JP 2008-500961 A (SEATTLE GENETICS INC.) 17 January 2008 (2008-01-17), entire text, all drawings | 1-56 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13.08.2021 | 24.08.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/026784

C (Continuation). DOCUMENTS CONSIDERED TO BE RELEVANT

| Category* | Citation of document, with indication, where appropriate, of the relevant passages | Relevant to claim No. |
|---|---|---|
| A | SARRUT, Morgan et al. Analysis of antibody-drug conjugates by comprehensive on-line two-dimensional hydrophobic interaction chromatography x reversed phase liquid chromatography hyphenated to high resolution mass spectrometry. II-Identification of sub-units for the characterization of even and odd load drug species, Journal of Chromatography B, 2016, 1032, pp. 91-102, entire text, all drawings | 1-56 |

Form PCT/ISA/210 (continuation of second sheet) (January 2015)

**INTERNATIONAL SEARCH REPORT**
Information on patent family members

International application No.
PCT/JP2021/026784

| Patent Documents referred to in the Report | Publication Date | Patent Family | Publication Date |
|---|---|---|---|
| WO 2017/002776 A1 | 05.01.2017 | US 2018/0147292 A1<br>entire text, all drawings<br>EP 3315512 A1<br>KR 10-2018-0021723 A<br>CN 107922477 A | |
| WO 2020/022363 A1 | 30.01.2020 | (Family: none) | |
| JP 2008-500961 A | 17.01.2008 | US 2009/0010945 A1<br>entire text, all drawings<br>US 2011/0097322 A1<br>WO 2005/084390 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005084390 A
- WO 2017002776 A
- US 6214345 B
- US 2002083067 A
- US 2003026577 A
- US 2004054622 A
- US 2005112919 A
- US 2006135371 A
- US 2007112193 A
- US 2008033891 A
- US 2009100194 A
- US 2009134976 A
- US 2009134977 A
- US 2010093395 A
- US 2011130613 A
- US 2011130616 A
- US 2013055993 A
- US 2014057687 A
- US 2014107024 A
- US 2014134457 A
- US 2014145090 A
- US 2015098099 A
- WO 2014057687 A
- WO 2015098099 A
- WO 9007861 A
- US 5821337 A
- WO 9954342 A
- WO 0061739 A
- WO 0231140 A
- WO 2019044947 A


### Non-patent literature cited in the description


- **DUCRY, L. et al.** *Bioconjugate Chem.,* 2010, vol. 21, 5-13
- **ALLEY, S. C. et al.** *Current Opinion in Chemical Biology,* 2010, vol. 14, 529-537
- **DAMLE N. K.** *Expert Opin. Biol. Ther.,* 2004, vol. 4, 1445-1452
- **SENTER P. D. et al.** *Nature Biotechnology,* 2012, vol. 30, 631-637
- **HOWARD A. et al.** *J Clin Oncol,* vol. 29, 398-405
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- **KOHLER ; MILSTEIN.** *Nature,* 1975, vol. 256, 495-497
- Monoclonal Antibodies. Plenum Press, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology:Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science.,* 2002, vol. 43 (7), 2301-2308
- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- *Protein Science,* 1995, vol. 4, 2411-2423